# EUROPÄISCHE PATENTSCHRIFT

(11) **EP 1 232 279 B1**
(45) Veröffentlichungstag und Bekanntmachung des Hinweises auf die Patenterteilung: **02.03.2005**
(21) Anmeldenummer: 00987237.5
(22) Anmeldetag: 13.11.2000
(51) Int. Cl.: C12P 41/00, C12P 13/00, C07C 213/08, C07C 213/10, C07C 217/10, C07C 215/08, C07C 235/08, C07B 57/00

(54) **VERFAHREN ZUR HERSTELLUNG OPTISCH AKTIVER AMINE**
METHOD FOR PRODUCING OPTICALLY ACTIVE AMINES
PROCEDE DE PREPARATION D'AMINES OPTIQUEMENT ACTIVES

(30) Priorität: 25.11.1999 DE 19956786
(43) Veröffentlichungstag der Anmeldung: 21.08.2002
(73) Patentinhaber: BASF AKTIENGESELLSCHAFT, 67056 Ludwigshafen (DE)
(72) Erfinder: DITRICH, Klaus, 67161 Gönnheim (DE); SIEGEL, Wolfgang, 67117 Limburgerhof (DE)
(86) Internationale Anmeldenummer: PCT/EP2000/011191
(87) Internationale Veröffentlichungsnummer: WO 2001/038292

(56) Entgegenhaltungen:
- WO-A-96/23894
- WO-A-97/28271
- DE-C- 247 144
- DE-C- 723 498
- CHEMICAL ABSTRACTS, vol. 41, no. 19, 10. Oktober 1947 (1947-10-10) Columbus, Ohio, US; abstract no. 6199d, O. SCHNIDER : "Synthesis of panthenol and its transformation into pantothenic acid." Seite 6199; Spalte 1; XP002162975 & JUBILEE VOL. EMIL BARELL, 1946, Seiten 79-84,
- ANDRES C ET AL: "STEREOSELECTIVE RING OPENING OF CHIRAL OXAZOLIDINES BY REFORMATSKY REAGENTS: AN ENANTIOSELECTIVE ENTRY TO BETA-AMINO ESTERS" TETRAHEDRON LETTERS,NL,ELSEVIER SCIENCE PUBLISHERS, AMSTERDAM, Bd. 33, Nr. 20, 12. Mai 1992 (1992-05-12), Seiten 2895-2898, XP002043792 ISSN: 0040-4039
- BESSE P ET AL: "Stereoselective chemoenzymatic synthesis of both enantiomers of protected 4-amino-2-pentanone" TETRAHEDRON: ASYMMETRY,NL,ELSEVIER SCIENCE PUBLISHERS, AMSTERDAM, Bd. 10, Nr. 11, 4. Juni 1999 (1999-06-04), Seiten 2213-2224, XP004174104 ISSN: 0957-4166
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 8253393 (BRN), XP002171088 & J. MED. CHEM., Bd. 42, Nr. 5, 1999, Seiten 833-848,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 1746407 (BRN), XP002171089 & J. ORG. CHEM., Bd. 14, 1949, Seite 702
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 1734381 (BRN), XP002171090 & MONATSH. CHEM., Bd. 91, 1960, Seiten 363-367,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 2231657 (BRN), XP002171091 & RUSS. CHEM. BL. , Bd. 46, Nr. 11, 1997, Seiten 1936-1938,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 7174696 (BRN), XP002171092 & J. MED. CHEM., Bd. 23, Nr. 2, 1980, Seiten 121-127,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 1732216; 1732706 (BRN), XP002171093 & J. AM. PHARM. ASSOC., Bd. 41, 1952, Seiten 328-332,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 5561939 (BRN), XP002171094 & J. ORG. CHEM. , Bd. 51, Nr. 25, 1986, Seiten 4905-4910,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 1719513 (BRN), XP002171095 & HEL. CHIM. ACTA, Bd. 38, 1955, Seite 915
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 4304559 (BRN), XP002171096 & SYNTH. COMMUN., Bd. 21, Nr. 1, 1991, Seiten 1-9,
- DATABASE CROSSFIRE BEILSTEIN [Online] BEILSTEIN INSTITUT ZUR FOEDERUNG DER CHEMISCHEN WISSENSCHAFTEN, FRANKFURT AM MAIN, DE; Database-Accession no. 1919664 (BRN), XP002171097 & CHEM. PHARM. BULL., Bd. 18, 1970, Seiten 1731-1736,
- MORRISON R.T. ET AL: 'LERHBUCH DER ORGANISCHEN CHEMIE', Bd. 3, VCH Seiten 1324 - 1325
- MARCH J.: 'ADVANCED ORGANIC CHEMISTRY', 1992, JOHN WILEY & SONS Seiten 795 - 797

## Beschreibung

Die vorliegende Erfindung betrifft ein Verfahren zur Herstellung optisch aktiver Amine. Weiterhin betrifft die Erfindung ein Verfahren zur Herstellung racemischen Amine, die mit optisch aktiven Carbonsäuren oder Enzymen racematgespalten werden können. Die Erfindung betrifft außerdem optisch aktive Amide.

Die Racematspaltung von Aminen durch Enzym-katalysierte Umsetzung mit Estem ist ebenso bekannt, wie die klassisch chemische Racematspaltung über die Bildung von diastereomeren Salzen mit optisch aktiven Carbonsäuren (Advanced Organic Chemistry, Reactions, Mechanisms and Structure, Jerry March, Fourth Edition, John Wiley & Sons, Inc., 1992, ISBN 0-471-60180-2). So beschreibt Kitaguchi et al. (J. Amer. Chem. Soc. 111, 3094-3095, 1989) beispielsweise die Racematspaltung von Aminen mit Trifluorethylbutyrat unter Subtilisin-Katalyse. Die Enantioselektivität dieser Reaktion ist jedoch stark lösungsmittelabhängig. Selbst mit dem geeignetsten der beschriebenen Lösungsmittel (3-Methyl-3-pentanol) wird nur eine mäßige Selektivität erreicht.

In WO 91/19002 wird ein Verfahren zur chiralen Anreicherung von asymmetrischen primären Aminen beschrieben, bei dem die Amine unter Subtilisin-Katalyse mit Ethylacetat oder Ethylbutyrat umgesetzt werden. Die dabei erzielten Enantiomerenüberschüsse sind jedoch nicht befriedigend; außerdem werden lange Reaktionszeiten von einer bis mehreren Wochen benötigt.

Gotor et al. (J. Chem. Soc. Chem. Commun. 957-958, 1988) beschreiben die enantioselektive Acylierung von 2-Amino-butan-1-ol mit Ethylacetat unter Katalyse von Schweinepankreas-Lipase (PPL). Dabei wird der verwendete Ester (Ethylacetat) auch als Lösungsmittel eingesetzt. Bei Verwendung anderer Lösungsmittel bzw. anderer Enzyme werden keine befriedigenden Ergebnisse erzielt.

Von Brieva et al. (J. Chem. Soc. Chem. Commun., 1386-1387, 1990) wird die enantioselektive Synthese von Amiden aus racemischen primären Aminen durch Umsatz mit 2-Chlorpropionat unter Katalyse von Subtilisin in Hexan oder Candida cylindracea Lipase in 3-Methyl-3-pentanol beschrieben.

Quiros et al. (Tetrahedron: Asymmetry 4, 1105-1112, 1993) beschreiben die Lipasekatalysierte Synthese von optisch aktiven Amiden aus racemischen a-Halogensubstituierten Propionsäureethylestern und primären Aminen. Die bei dieser Umsetzung erreichte Enantioselektivität kann jedoch nicht zufriedenstellen.

Asensio et al. (Tetrahedron Letters 32, 4197-4198, 1991) beschreiben die Lipasekatalysierte enantioselektive Acylierung von sekundären Aminen. Diese Reaktion verläuft jedoch nur bei einem Amin enantioselektiv und auch dort nur mit mäßigem Erfolg. Andere Amine zeigen überhaupt keine Enantioselektivität.

In US 5,057,607 wird die N-Acylierung von 3-Amido-azetdionverbindungen mit Hilfe der Penicillin G-Amidase beschrieben. Die Penicillin G-Amidase hat jedoch nur ein sehr eingeschränktes Substratspektrum, so daß sie nur für die Herstellung von b-Lactamen verwendet werden kann.

In WO 95/08636 wird ein Verfahren zur Racematspaltung von primären und sekundären Aminen durch Enzym-katalysierte Acylierung beschrieben. Weitere Schriften, die Verfahren zur Enzym-katalysierten Racematspaltung von Aminen beschreiben, sind beispielsweise WO 96/23894, WO 97/20946, WO 97/2871, WO 97/46698 und WO 98/03465.

In den oben genannten Schriften werden verschiedene Methoden zur Racematspaltung von Aminen beschrieben. Als Produkt der Racematspaltung wird neben dem eigentlichen Wertprodukt auch immer 50 % des unerwünschten Enantiomers gebildet. Für eine wirtschaftliche Nutzung dieser Verfahren ist es wichtig, daß dieses unerwünschte Enantiomer racemisiert und in den Racematspaltungsprozeß rückgeführt werden kann oder aber daß dieses zunächst unerwünschte Enantiomer ebenfalls eine für chemische Synthesen gesuchte Verbindung und damit ein Wertprodukt ist. Bei der enzymatischen Racematspaltung fällt eines der Enantiomere in Form des Amides an. Ist dieses Amid das Wertprodukt, so muß es unter Erhalt des Stereozentrums gespalten werden. Ein derartiges Verfahren zur Spaltung optisch aktiver Amide unter Erhalt des Stereozentrums wird beispielsweise in US 5,905,167 beschrieben.

Ein wesentliches Problem für die technische Racematspaltung optisch aktiver funktionalisierter Amine wie Aminoalkohole ist nach wie vor die Bereitstellung des racemischen Aminoalkohols, der als Ausgangsmaterial für die Racematspaltung verwendet wird. Für ein wirtschaftliches Verfahren ist es erforderlich, daß eine einfache, sichere und kostengünstige Synthese zum racemischen funktionalisierten Amin zur Verfügung steht. Von Murata et al. (J. Chem. Soc. Jpn, Ind. Chem. Sect. 56, 1953: 628, Chem. Abstr. 49, 1955, 7517g) wird ein Verfahren zur Herstellung von 1-Benzyloxy-3-butanon aus 3-Buten-2-on unter Verwendung von 3 Äquivalenten Benzylalkohol und 0,04 mol% Na-Methanolat beschrieben. Von Nachteil bei dieser Methode ist, daß der überschüssige Benzylalkohol über einen weiteren Verfahrensschritt abgetrennt werden muß. Außerdem kommt es bei der Ausführung des Verfahrens immer wieder zur Rückspaltung des Produkts in die Ausgangsmaterialien, das heißt es fehlt an der für ein technisches Verfahren notwendigen Prozeßsicherheit.

Die bisher bekannten Verfahren zur Racematspaltung und Bereitstellung der racemischen funktionalisierten Amine haben den Nachteil, daß sie nicht die für eine technische Nutzung erforderliche Einfachheit und Prozeßsicherheit haben und damit nur unter ganz speziellen Bedingungen durchgeführt werden können. Außerdem benötigen sie eine erhebliche Mengen an Ausgangsmaterialien für die Synthese des racemischen Edukts, so daß ein darauf aufbauendes Verfahren nicht wirtschaftlich ist.

Es bestand daher die Aufgabe, ein Verfahren zur Synthese racemischer funktionalisierter Amine wie Aminoalkohole sowie ein darauf aufbauendes Verfahren zur Racematspaltung der racemischen Amine bereitzustellen, das eine hohe Prozessicherheit bei hoher Enantioselektivität in der Racematspaltung gewährleistet und in einem weiten Bereich von Reaktionsbedingungen eingesetzt werden kann und dabei mit möglichst geringen Mengen an Edukt und Katalysator auskommt, so daß die Kosten des Gesamtverfahrens weiter gesenkt werden können.

Diese Aufgabe wurde gelöst durch ein Verfahren zur Herstellung von Verbindungen der allgemeinen Formel I 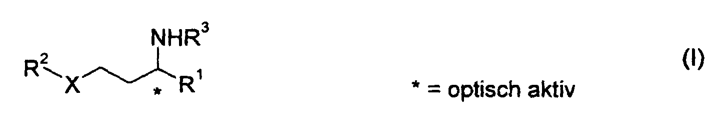 dadurch gekennzeichnet, daß es folgende Verfahrensschritte umfaßt:
a) Umsetzung von Verbindungen der allgemeinen Formel II 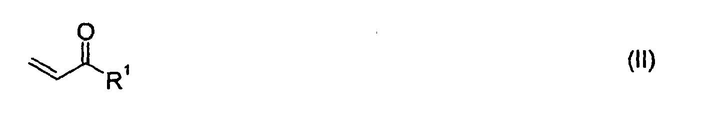 mit Verbindungen der Formel R²-XH (III) in Gegenwart einer Base zu Verbindungen der allgemeinen Formel IV 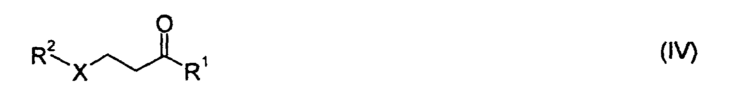
b) Umsetzung der Reaktionslösung von Verbindungen der allgemeinen Formel IV mit einer Verbindung der allgemeinen Formel NH₂R³ (V) zu Verbindungen der allgemeinen Formel VI 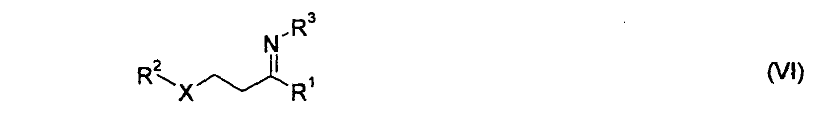
c) Hydrierung von Verbindungen der allgemeinen Formel VI zu Verbindungen der allgemeinen Formel VII 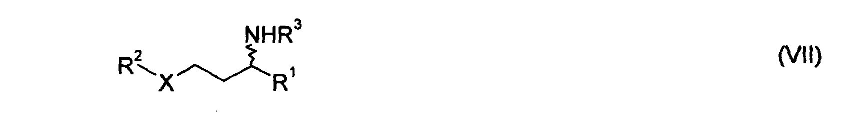 in Gegenwart eines Hydrierkatalysators,
d) Racematspaltung von Verbindungen der allgemeinen Formel VII zu Verbindungen der allgemeinen Formel I mit einer optisch aktiven Carbonsäure oder mit Estern der allgemeinen Formel VIII 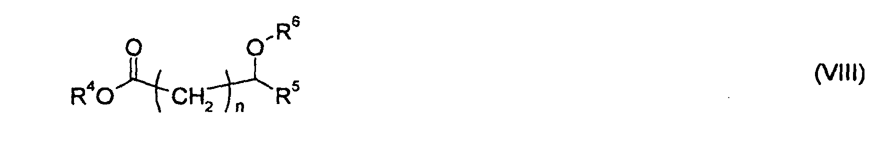 in Gegenwart einer Lipase oder Esterase, wobei die Substituenten und Variablen in den Formeln I, II, III, IV, V, VI, VII und VIII folgende Bedeutung haben:
   - R¹: substituiertes oder unsubstituiertes, verzweigtes oder unverzweigtes C₁-C₁₀-Alkyl-, Arylalkyl-, Aryl-, Hetarylalkyl- oder Hetaryl-,
   - R²: substituiertes oder unsubstituiertes, verzweigtes oder unverzweigtes C₁-C₁₀-Alkyl-, Arylalkyl-, Aryl-, Hetarylalkyl- oder Hetaryl-,
   - R³: Wasserstoff, Hydroxyl-, substituiertes oder unsubstituiertes, verzweigtes oder unverzweigtes C₁-C₁₀-Alkyl-,
   - R⁴: substituiertes oder unsubstituiertes, verzweigtes oder unverzweigtes C₁-C₁₀-Alkyl-,
   - R⁵: Wasserstoff, substituiertes oder unsubstituiertes, verzweigtes oder unverzweigtes C₁-C₁₀-Alkyl-,
   - R⁶: Wasserstoff, substituiertes oder unsubstituiertes, verzweigtes oder unverzweigtes C₁-C₁₀-Alkyl- oder substituiertes oder unsubstituiertes Phenyl,
   X = Sauerstoff oder Stickstoff, bevorzugt Sauerstoff
   n = 0 oder 1.

R¹ bezeichnet in den Verbindungen der Formeln I, II, IV, VIund VII substituiertes oder unsubstituiertes, verzweigtes oder unverzweigtes C₁-C₁₀-Alkyl-, Arylalkyl-, Aryl-, Hetarylalkyl- oder Hetaryl-. Bevorzugte Reste von R¹ sind substituiertes oder unsubstituiertes, verzweigtes oder unverzweigtes C₁-C₁₀-Alkyl- oder Aryl-.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl oder Propyl.

Als Substituenten der oben genannten Reste von R¹ kommen Substituenten in Frage wie ein oder mehrere Substituenten wie Halogen wie Fluor, Amino, Hydroxy, Benzyloxy, Phenyl oder Benzyl.

Als Arylalkylreste seien verzweigtkettige oder unverzweigtkettige Phenyl-(C₁-C₅-Alkyl)-oder Naphthyl-(C₁-C₅-Alkyl)-Reste wie Phenylmethyl, Phenylethyl, Phenylpropyl, Phenyl-1-methylethyl, Phenylbutyl, Phenyl-1-methylpropyl, Phenyl-2-methylpropyl, Phenyl-1,1-dimethylethyl, Phenylpentyl, Phenyl-1-methylbutyl, Phenyl-2-methylbutyl, Phenyl-3-methylbutyl, Phenyl-2,2-dimethylpropyl, Phenyl-1-ethylpropyl, Naphthylmethyl, Naphthylethyl, Naphthylpropyl, Naphthyl-1-methylethyl, Naphthylbutyl, Naphthyl-1-methylpropyl, Naphthyl-2-methylpropyl, Naphthyl-1,1-dimethylethyl-, Naphthylpentyl, Naphthyl-1-methylbutyl, Naphthyl-2-methylbutyl, Naphthyl-3-methylbutyl, Naphthyl-2,2-dimethylpropyl, oder Naphthyl-1-ethylpropyl, sowie ihre isomeren oder stereoisomeren Formen genannt. Bevorzugte Reste sind verzweigtkettige oder unverzweigtkettige Phenyl- (C₁-C₅-Alkyl)-Reste wie wie Phenylmethyl, Phenylethyl oder Phenylpropyl.

Als Arylreste seien beispielsweise Phenyl, Methoxyphenyl oder Naphthyl, oder aromatische Ringe oder Ringsysteme mit 6 bis 18 Kohlenstoffatomen im Ringsystem sowie bis zu 24 weiteren C-Atomen, die weitere nicht aromatische Ringe oder Ringsysteme mit 3 bis 8 C-Atomen im Ring bilden können, zu verstehen, die ggf. mit einem oder mehreren Resten wie Halogen wie Fluor, Amino, Hydroxy, Alkyl, Alkoxy oder anderen Resten substituiert sein können, genannt. Bevorzugt sind ggf. substituiertes Phenyl, Methoxyphenyl oder Naphthyl.

Als Hetaryl(alkyl)reste seien beispielsweise Hetarylaikylreste, die ein oder mehrere Stickstoff-, Schwefel- und/oder Sauerstoffatome im Ring oder Ringsystem enthalten und mit einer verzweigten oder unverzweigten C₁-C₅-Alkylenkette wie Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen oder 1-Ethylpropylen verbunden sind, genannt.

Als Hetarylreste seien einfache oder kondensierte aromatische Ringsysteme mit einem oder mehreren heteroaromatischen 3- bis 7gliedrigen Ringen, die ein oder mehrere Heteroatome wie N, O oder S enthalten können, und die ggf. mit einem oder mehreren Resten wie Halogen wie Fluor, Amino, Hydroxy, Thio, Alkyl, Alkoxy oder weiteren aromatischen oder weiteren gesättigten oder ungesättigten nicht aromatischen Ringen oder Ringsystemen substituiert sein können, genannt.

R² bezeichnet in den Verbindungen der Formeln I, III, IV, VI und VII substituiertes oder unsubstituiertes, verzweigtes oder unverzweigtes C₁-C₁₀-Alkyl-, Arylalkyl-, Aryl-, Hetarylalkyl- oder Hetaryl-. Bevorzugte Reste von R' sind substituiertes oder unsubstituiertes, verzweigtes oder unverzweigtes C₁-C₁₀-Alkyl- oder Arylalkyl-.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl oder Propyl.

Als Substituenten der oben genannten Reste von R² kommen prinzipiell alle denkbaren Substituenten in Frage beispielsweise ein oder mehrere Substituenten wie Halogen wie Fluor, Amino, Hydroxy, Benzyloxy, Phenyl oder Benzyl.

Als Arylalkylreste seien verzweigtkettige oder unverzweigtkettige Phenyl-(C₁-C₅-Alkyl)-oder Naphthyl-(C₁-C₅-Alkyl)-Reste wie Phenylmethyl, Phenylethyl, Phenylpropyl, Phenyl-1-methylethyl, Phenylbutyl, Phenyl-1-methylpropyl, Phenyl-2-methylpropyl, Phenyl-1,1-dimethylethyl, Phenylpentyl, Phenyl-1-methylbutyl, Phenyl-2-methylbutyl, Phenyl-3-methylbutyl, Phenyl-2,2-dimethylpropyl, Phenyl-1-ethylpropyl, Naphthylmethyl, Naphthylethyl, Naphthylpropyl, Naphthyl-1-rnethylethyl, Naphthylbutyl, Naphthyl-1-methylpropyl, Naphthyl-2-methylpropyl, Naphthyl-1,1-dimethylethyl, Naphthylpentyl, Naphthyl-1-methylbutyl, Naphthyl-2-methylbutyl, Naphthyl-3-methylbutyl, Naphthyl-2,2-dimethylpropyl, oder Naphthyl-1-ethylpropyl, sowie ihre isomeren oder stereoisomeren Formen genannt. Bevorzugte Reste sind Phenylmethyl-, Phenylethyl- oder Naphthylmethyl.

Als Arylreste seien beispielsweise Phenyl, Methoxyphenyl oder Naphthyl, oder aromatische Ringe oder Ringsysteme mit 6 bis 18 Kohlenstoffatomen im Ringsystem sowie bis zu 24 weiteren C-Atomen, die weitere nicht aromatische Ringe oder Ringsysteme mit 3 bis 8 C-Atomen im Ring bilden können, zu verstehen, die ggf. mit einem oder mehreren Resten wie Halogen wie Fluor, Amino, Hydroxy, Alkyl, Alkoxy oder anderen Resten substituiert sein können, genannt. Bevorzugt sind ggf. substituierte Phenyl, Methoxyphenyl oder Naphthyl.

Als Hetaryl(alkyl)reste seien beispielsweise Hetarylalkylreste, die ein oder mehrere Stickstoff-, Schwefel- und/oder Sauerstoffatome im Ring oder Ringsystem enthalten und mit einer verzweigten oder unverzweigten C₁-C₅-Alkylenkette wie Methylen, Ethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen oder 1-Ethylpropylen verbunden sind, genannt.

Als Hetarylreste seien einfache oder kondensierte aromatische Ringsysteme mit einem oder mehreren heteroaromatischen 3- bis 7gliedrigen Ringen, die ein oder mehrere Heteroatome wie N, O oder S enthalten können, und die ggf. mit einem oder mehreren Resten wie Halogen wie Fluor, Amino, Hydroxy, Thio, Alkyl, Alkoxy oder weiteren aromatischen oder weiteren gesättigten oder ungesättigten nicht aromatischen Ringen oder Ringsystemen substituiert sein können, genannt.

R³ bezeichnet in den Verbindungen der Formeln I, V, VI, VII und IX Wasserstoff, Hydroxyl-, substituiertes oder unsubstituiertes, verzweigtes oder unverzweigtes C₁-C₁₀-Alkyl-. Bevorzugte Reste sind Wasserstoff oder Hydroxyl-.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl oder Propyl.

Als Substituenten der oben genannten Reste von R³ kommen prinzipiell alle denkbaren Substituenten in Frage beispielsweise ein oder mehrere Substituenten wie Halogen wie Fluor, Amino, Hydroxy, Benzyloxy, Phenyl oder Benzyl.

R⁴ bezeichnet in den Verbindungen der Formeln VIII substituiertes oder unsubstituiertes, verzweigtes oder unverzweigtes C₁-C₁₀-Alkyl-.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethytpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl, 1-Methylethyl, Propyl, Butyl oder Pentyl.

Als Substituenten der oben genannten Reste von R⁴ kommen prinzipiell alle denkbaren Substituenten in Frage beispielsweise ein oder mehrere Substituenten wie Halogen wie Fluor, Chlor oder Brom, Cyano, Nitro, Amino, Hydroxy, Alkyl, Cycloalkyl, Aryl, Alkoxy, Benzyloxy, Phenyl oder Benzyl. Bevorzugte Substituenten sind Halogen wie Chlor oder Brom, Cyano, Benzyloxy, C1-C4-Alkyl- oder Hydroxy.

R⁵ bezeichnet in den Verbindungen der Formeln VIII und IX Wasserstoff, substituiertes oder unsubstituiertes, verzweigtes oder unverzweigtes C₁-C₁₀-Alkyl-.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Wasserstoff, Methyl, Ethyl oder Propyl.

Als Substituenten der oben genannten Reste von R⁵ kommen prinzipiell alle denkbaren Substituenten in Frage beispielsweise ein oder mehrere Substituenten wie Halogen wie Fluor, Chlor oder Brom, Cyano, Nitro, Amino, Hydroxy, Benzyloxy, Phenyl oder Benzyl.

R⁶ bezeichnet in den Verbindungen der Formeln VIII und IX Wasserstoff, substituiertes oder unsubstituiertes, verzweigtes oder unverzweigtes C₁-C₁₀-Alkyl- oder substituiertes oder unsubstituiertes Phenyl.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl oder Propyl.

Als Substituenten der oben genannten Reste von R⁶ kommen prinzipiell alle denkbaren Substituenten in Frage beispielsweise ein oder mehrere Substituenten wie Halogen wie Fluor, Chlor oder Brom, Cyano, Nitro, Amino, Hydroxy, Benzyloxy, Phenyl oder Benzyl.

Die Erfindung betrifft weiterhin ein Verfahren zur Herstellung von Verbindungen der allgemeinen Formel VII 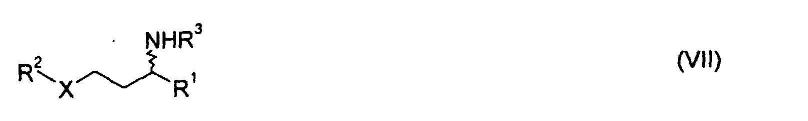 dadurch gekennzeichnet, daß es folgende Verfahrensschritte umfaßt:
a) Umsetzung von Verbindungen der allgemeinen Formel II 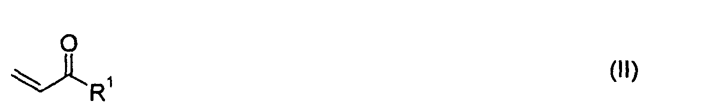 mit Verbindungen der Formel R²-XH (III) in Gegenwart einer Base zu Verbindungen der allgemeinen Formel IV 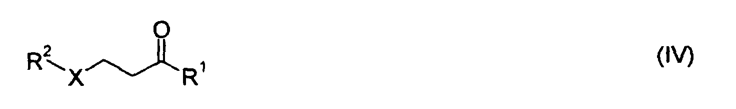
b) Umsetzung der Reaktionslösung von Verbindungen der allgemeinen Formel IV mit einer Verbindung der allgemeinen Formel NH₂R³ (V) zu Verbindungen der allgemeinen Formel VI 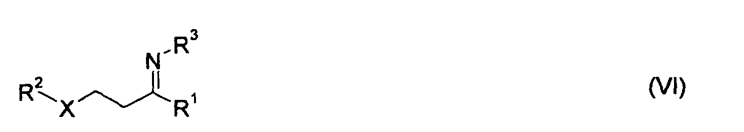
c) Hydrierung von Verbindung der allgemeinen Formel VI zu Verbindungen der allgemeinen Formel VII in Gegenwart eines Hydrierkatalysators, wobei die Substituenten und Variablen in den Formeln II, III, IV, V, VI und VII die gemäß Anspruch 1 genannte Bedeutung haben.

Schema I gibt die erfindungsgemäßen Verfahren zur besseren Übersicht beispielhaft wieder. Die Verfahrensschritte (a) bis (d) entsprechen dem Verfahren zur Herstellung funktionalisierter optisch aktiver Amine beispielsweise optisch aktiver Aminoalkohole oder optisch aktiver Diamine. Die Verfahrensschritte (a) bis (c) entsprechen dem Verfahren zur Herstellung funktionalisierter racemischer Amine, die anschließend einer Racematspaltung unterzogen werden können. In Schema I wird diese Racematspaltung am Beispiel der enzymatischen Racematspaltung gezeigt. Sie kann aber auch über andere Verfahren zur Racematspaltung vorgenommen werden wie die klassisch chemische Racematspaltung über diastereomere Salze, oder über chromatographische Verfahren. Die in Schema I bezeichneten Substituenten und Variablen haben die oben genannte Bedeutung. Das Schema gibt außerdem die optionale Möglichkeit der Neutralisierung der unter Schritt (a) erhaltenen Reaktionslösung vor der Umsetzung mit Verbindungen der allgemeinen Struktur bzw. Formel NH₂R³ (V) wieder. Werden Verbindungen der allgemeinen Formel R₂-XH (III) in einem großen Überschuß bezogen auf den Reaktionspartner (II) eingesetzt, so ist eine Neutralisierung der Reaktionslösung vor der weiteren Umsetzung erforderlich, da es sonst bei der Abreicherung der überschüssigen Verbindung R₂-XH zur unerwünschten Rückspaltung des Reaktionsproduktes kommt. Dies führt zu Ausbeuteverlusten und macht eine Aufreinigung des Produktes erforderlich. Bei Verwendung der erfindungsgemäßen vorteilhaften Menge der Verbindung (III) von 0,5 bis 2,5 Äquivalenten, bevorzugt 0,5 bis 2,0; besonders bevorzugt von 0,7 bis 1,5; ganz besonders bevorzugt von 0,9 bis 1,1 Äquivalenten bezogen auf den Reaktionspartner (II) ist eine Abreicherung nicht erforderlich und eine Neutralisation der Reaktionslösung vor dem Verfahrensschritt b in den erfindungsgemäßen Verfahren vorteilhafterweise nicht erforderlich. Sie kann jedoch je nach Edukten vorteilhaft vorgenommen werden, um die Verfahrenssicherheit zu erhöhen. Zur Neutralisation können alle üblichen Mineralsäuren wie HCl, H₂SO₄ oder H₃PO₄ oder organische Säuren wie niedere aliphatische Carbonsäuren wie Ameisensäure verwendet werden. Bevorzugt wird ortho-Phosphorsäure verwendet.

Als Base für das erfindungsgemäße Verfahren in Verfahrensschritt (a) sind prinzipiell alle Basen geeignet, die die Addition der Verbindung (III) an das Michaelsystem der Verbindung (II) katalysieren können wie NaOH, KOH, tertiäre Amine oder Alkali- und Erdalkalialkoholate. Als vorteilhafte Basen seien beispielhaft Alkali- und Erdalkalialkoholate wie Natriummethanolat, Natriumethanolat, Natriumbutanolat, Natrium-tert.butanolat, Kalium-tert.-butanolat oder starke basische Amine wie Diazabycycloundecen genannt. Die Base wird als Katalysator vorteilhaft in einer Konzentration von 0,001 bis 10 mol%, bevorzugt von 0,01 bis 5 mol%, besonders bevorzugt von 0,3 bis 0,5 mol% bezogen auf die eingesetzte Verbindung (III) verwendet.

Verfahrensschritt (a) der erfindungsgemäßen Verfahren kann in Gegenwart eines unter Reaktionsbedingungen inerten, aprotischen Lösungsmittels durchgeführt werden. Beispiele für Lösungsmittel sind Kohlenwasserstoffe wie Hexan, Cyclohexan, Benzol oder Toluol oder Ether wie Methyltertiärbutylether (= MTBE), Diethylether, Dibutylether oder Tetrahydrofuran (= THF). Vorteilhaft wird die Reaktion in Abwesenheit eines Lösungsmittels durchgeführt.

Die Reaktion [Verfahrensschritt (a)] wird vorteilhaft bei einer Temperatur von -30 bis +50°C, bevorzugt von -5 bis + 10°C durchgeführt.

Vorteilhafte Verbindungen der allgemeinen Formel (V) sind neben Ammoniak, primäre und sekundäre aliphatische Amine und Hydroxylamin. Vorteilhaft wird Verfahrensschritt (b) mit Hydroxylamin durchgeführt, das als wäßrige Lösung, oder als wäßrige Lösung seines Salzes wie z.B. Hydroxylamin-hydrochlorid, Hydroxylamin-sulfat gegebenenfalls wie auch die anderen Verbindungen der Formel (V) in Anwesenheit einer Base wie Natronlauge oder Natriumacetat eingesetzt wird. Bevorzugt wird eine wäßrige Lösung des freien Hydroxylamins verwendet. Die Reaktion kann vorteilhaft in einem inerten, protischen Lösungsmittel wie Wasser oder einem Alkohol wie Methanol, Ethanol, Propanol, Butanol oder iso-Butanol durchgeführt werden. Bevorzugt wird die Reaktion in Wasser durchgeführt.

Die Reaktion [Verfahrensschritt (b)] wird vorteilhaft bei einer Temperatur von 0 bis + 100°C, bevorzugt von 20 bis 40°C durchgeführt.

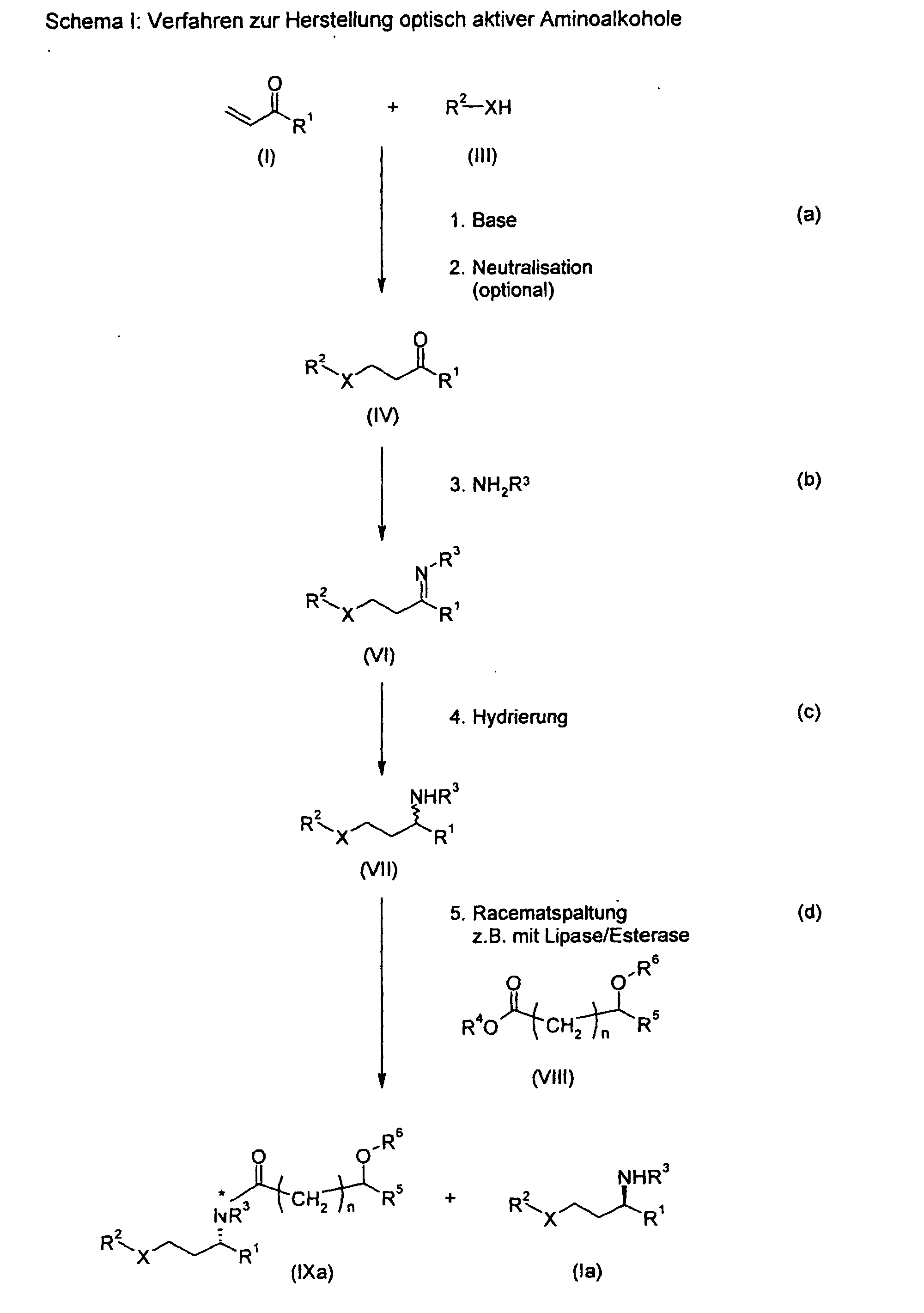

Die Hydrierung in den erfindungsgemäßen Verfahren [Verfahrensschritt (c)] kann mit allen üblichen Hydrierkatalysatoren beispielsweise auf Basis von Ni, Co, Hg, Pt, Pd, Ru oder Rh durchgeführt werden. Ein üblicher für die homogene Katalyse verwendeter Katalysator ist beispielsweise der Wilkinson-Katalysator. Die Hydrierung kann in heterogener oder homogener Katalyse erfolgen. Aus wirtschaftlichen Gründen und wegen der guten Verfügbarkeit wird Raney-Ni#kel als Katalysator bevorzugt. Die Hydrierung wird vorteilhaft in einem unter den Reaktionsbedingungen inerten Lösungsmittel durchgeführt. Derartige Lösungsmittel sind beispielsweise Kohlenwasserstoffe wie Hexan, Cyclohexan, Benzol oder Toluol, Ether wie MTBE, Diethylether, Dibutylether oder THF, oder Alkohole wie Methanol, Ethanol, Propanol, Butanol oder iso-Butanol. Bevorzugt werden als Lösungsmittel THF oder Methanol verwendet.

Die Reaktion [Verfahrensschritt (c)] wird vorteilhaft bei einer Temperatur von 0 bis +150°C, bevorzugt von 50 bis 100°C durchgeführt. Die Hydrierung wird üblicherweise in einem Druckbereich von Normaldruck bis 300 bar durchgeführt. Bevorzugt wird die Reaktion bei einem Druck von 50 bis 150 bar durchgeführt.

Die Racematspaltung kann wie oben beschrieben über eine enzymatische oder klassisch chemische Racematspaltung erfolgen auch chromatographische Verfahren können verwendet werden. Bevorzugt wird die Racematspaltung mit Hilfe von Enzymen oder diastereomere Salze durchgeführt, besonders bevorzugt über Enzyme.

Für die Trennung über diastereomere Salze kommen prinzipiell alle optisch aktiven Carbonsäuren in Frage. Vorteilhafte optisch aktive Carbonsäuren sind Weinsäure, Di-benzoyl-Weinsäure, Mandelsäure, Camphersäure, Campersulfonsäure, p-Hydroxy-Mandelsäure, p-Cl-Mandelsäure, Phenoxypropionsäure, p-Hydroxy-phenoxy-propionsäure oder Milchsäure. Bevorzugt wird zur Racematspaltung Mandelsäure verwendet. Die Salzbildung kann in einem inerten Lösungsmittel erfolgen wie einem Kohlenwasserstoff wie Hexan, Cyclohexan, Benzol oder Toluol; oder einem Ether wie MTBE, Diethylether, Dibutylether oder THF; oder einem Alkohol wie Methanol, Ethanol, Propanol, iso-Propanol, Butanol oder iso-Butanol. Zum Umkristallisieren benutzt man zweckmäßigerweise einen Alkohol wie Methanol, Ethanol, Propanol, iso-Propanol, Butanol oder iso-Butanol. Bevorzugt wird iso-Propanol verwendet. Zur Verbesserung der Kristallisation kann die Lösung gekühlt werden. Im Falle, daß das gebildete Salz spontan ausfällt, wird es über Erhitzen wieder in Lösung gebracht und langsam unter Abkühlung wieder auskristallisiert. Falls erforderlich kann die Kristallisation mehrfach durchgeführt werden.

Die Racematspaltung der funktionalisierten Amine kann vorteilhaft auch über Enzyme wie Esterasen oder Lipasen durchgeführt werden.

Als Esterasen und Lipasen sind für das erfindungsgemäße Verfahren prinzipiell alle aus Pflanzen, Tieren oder Mikroorganismen verfügbaren Esterasen und Lipasen geeignet. Vorteilhaft sind mikrobielle Lipasen geeignet, die beispielsweise aus eukaryontischen Organismen wie Pilzen oder Hefen oder prokaryontischen Organismen wie Bakterien isolierbar sind. Besonders gut geeignet sind bakterielle Lipasen aus den Gattungen Bacillus oder Pseudomonas, z.B. Amano P oder die Lipase aus Pseudomonas spec. DSM 8246 oder Lipasen aus Pilzen wie Aspergillus oder aus Hefen wie Candida oder Yerrowia. Weitere vorteilhaft geeignete Lipasen sind beispielsweise von der Firma Novo Nordisk kommerziell erhältlichen Enzyme, insbesondere die Lipasen SP 523, SP 524, SP 525, SP 526 und Novozym® 435, die aus Hefen wie Candida antarctica gewonnen werden. Weitere Beispiele sind die von Roche Molecular Biochemicals (Roche Diagnostic GmbH, Penzberg) kommerziell erhältlichen Lipasen Chirazyme L1, L2, L3, L4, L5, L6, L7 und L8.

Die Lipasen können in nativer oder immobilisierter Form eingesetzt werden. Die immobilisierte Lipasen können mikroverkapselt werden, mit Präpolymerisaten emulgiert und polymerisiert sein, mit bifunktionellen Substanzen (Oligomeren, Aldehyden etc.) vernetzt sein oder an anorganische oder organische Trägermaterialien, wie z.B. Celite, Lewatit, Zeolithe, Polysaccharide, Polyamide oder Polystyrolharze, gebunden vorliegen. Besonders bevorzugt sind die Lipasen Novozym® 435 und Chirazyme L2.

Die Reaktion mit den Esterasen oder Lipasen findet im Allgemeinen bei Atmosphärendruck statt, gegebenenfalls unter Inertgas, wie Stickstoff oder Argon. Sie kann aber auch unter erhöhten Druck ausgeführt werden.

Die Temperatur für die Umsetzung der racemischen Amine der Formel VII mit den für die Racematspaltung geeigeneten Estern, die in alpha Position zum Carbonylkohlenstoff ein Sauerstoffatom tragen, speziell den Estern mit der allgemeinen Formel VIII 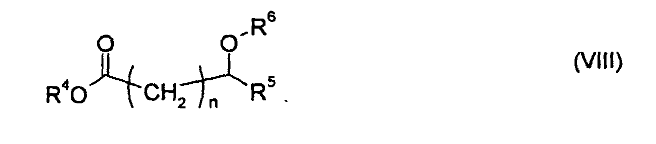 liegt üblicherweise bei 0 bis 90°C, bevorzugt bei 10 bis 60°C, besonders bevorzugt bei 20 bis 50°C. Die Substituenten der bevorzugten Ester VIII haben die oben genannte Bedeutung.

Die Umsetzung des Esters mit dem racemischen funktionalisierten Amin, das heißt dem entsprechenden Aminoalkohol oder Diamin unter Enzymkatalyse wird üblicherweise bei Raumtemperatur ausgeführt. Die Reaktionszeiten dafür betragen je nach Substrat 1 bis 48 Stunden. Sekundäre Aminoalkohole bzw. Diamine benötigen in der Regel längere Reaktionszeiten als primäre Aminoalkohole oder Diamine. Die geringere Reaktivität sekundärer Amine kann auch durch eine gegenüber primären Aminen erhöhte Menge an Katalysator ausgeglichen werden.

Pro Mol racemischen Amin werden 0,5 bis 2,0 Mol, bevorzugt 0,5 bis 1 Mol Ester eingesetzt. Die erforderliche Enzymmenge hängt von der Aktivität der Enzympräparation und der Reaktivität des Amins ab und kann leicht durch Vorversuche ermittelt werden. In der Regel werden 0,1 bis 10 Gew.-%, bevorzugt 1 bis 5 Gew.-% der immobilisierte Enzympräparation (bezogen auf das racemische Amin) eingesetzt. Novozym® 435 hat eine Aktivität von etwa 7000 PL U/g - 10000 PL U/g (PL = Propyllaurat Units, Units werden auf Propyllaurat als Substrat bezogen).

Die zuzusetzende Menge an Enzym hängt von der Art des Enzyms und der Aktivität der Enzympräparation ab. Die für die Reaktion optimale Enzymmenge kann leicht durch einfache Vorversuche ermittelt werden.

Der Reaktionsverlauf läßt sich leicht mit üblichen Methoden wie Gaschromatographie oder Hochdruckflüssigchromatographie verfolgen. Wenn der gewünschte Umsatz, in der Regel 50 %, erreicht ist, wird die Reaktion vorzugsweise durch Entfernen des Katalysators, beispielsweise durch Abfiltrieren des (geträgerten) Enzyms, beendet. Die Reaktion kann beispielsweise auch durch Zugabe von Enzym zerstörenden Substanzen wie Säuren oder Laugen oder durch Erhitzen gestoppt werden. Bei kontinuierlicher Fahrweise kann der Umsatz über die Belastung des Enzyms, d.h. der Menge Amin, die pro Zeiteinheit durch den Enzymreaktor gepumpt wird, geregelt werden. Das Verfahren kann vorzugsweise kontinuierlich geführt werden, aber auch batch-weise oder semikontinuierlich durchgeführt werden.

Die enzymkatalysierte Racematspaltung kann sowohl in protischen oder aprotischen Lösungsmitteln als auch lösungsmittelfrei durchgeführt werden. Geeignete Lösungsmittel sind z.B. Kohlenwasserstoffe wie Hexan, Cyclohexan oder Toluol, Ether wie z.B. Diethylether, Dioxan, Methyl-tert.-butylether, tert.-Amyl-methylether oder THF, Nitrile, wie Acetonitril, Butyronitril, Alkohole, wie tert.-Butanol, 3-Methyl-3-pentanol, und halogenierte Kohlenwasserstoffe wie z.B. Methylenchlorid.

Die Reaktion verläuft besonders gut, wenn die Lösungsmittel und Einsatzstoffe möglichst wasserfrei vorliegen. Vorteilhaft werden für die Racematspaltung die Lösungsmitttel und Einsatzstoffe Amin und Ester getrocknet. Dies kann prinzipiell in jeder dem Fachmann bekannten Weise erfolgen, z.B. durch azeotrope Trocknung oder durch Tro#kenmittel wie Natriumsulfat, Magnesiumsulfat, KOH, Phosphorpentoxid, Molekularsieb, Kieselgel oder Aluminiumoxid.

Am Ende der enzymkatalysierten Racematspaltung liegt ein Gemisch aus dem acylierten Amin-Enantiomer der allgemeinen Formel IX 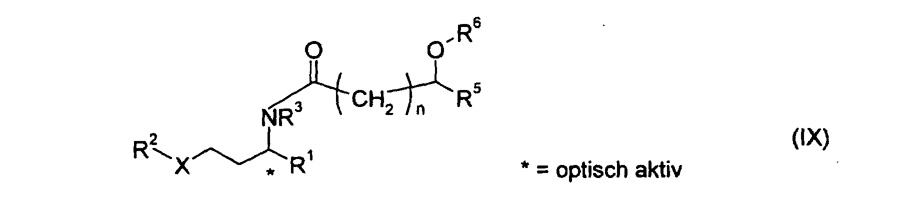 dem nicht umgesetztem Amin-Enantiomer, dem bei der Acylierung aus dem Ester freigesetzten Alkohol und evtl. im Überschuß eingesetztem Ester vor. Zur Trennung dieses Gemischs eignen sich insbesondere destillative und extraktive Verfahren. So können niedrig siedende Amine direkt aus dem Reaktionsgemisch abdestilliert werden. Das Amid läßt sich anschließend destillativ oder extraktiv vom Alkohol und gegebenenfalls Ester abtrennen und kann dann in üblicher Weise, beispielsweise durch Kochen mit Natron- oder Kalilauge, unter Racemisierung oder auch ohne Racemisierung (siehe US 5,905,167) hydrolysiert werden. US 5,905,167 beschreibt in den Spalten 2 bis 5 und in Beispiel 2 ein Verfahren zur Spaltung von Amiden unter Erhalt des Stereozentrums. Das bei der Hydrolyse gebildete zweite Amin-Enantiomer kann von der als Salz vorliegenden Carbonsäure destillativ oder extraktiv isoliert werden. Das geschieht bevorzugterweise durch Extraktion, wobei als Extraktionsmittel Ether wie Diethylether, Methyl-tert.-Butylether und Dibutylether, halogenierte Kohlenwasserstoffe wie Dichlormethan oder Trichlorethylen oder Kohlenwasserstoffe wie Pentan, Hexan, Cyclohexan, Benzol, Toluol und Xylol eingesetzt werden. Eine ebenfalls bevorzugte Ausführungsform der Isolierung des Amins ist die Wasserdampfdestillation. Eine besonders geeignete Ausführungsform der Erfindung besteht darin, daß man die Spaltung bei einer so hohen Temperatur ausführt, das das erhaltene Reaktionsprodukt (Amin) mit dem Wasserdampf überdestilliert und so gleich aus dem Reaktionsgemisch entfernt wird, während die Säure, die bei den alkalischen Bedingungen dissoziiert vorliegt, in der Vorlage zurückbleibt. Auf den genannten Wegen lassen sich prinzipiell Amine in den erfindungsgemäßen Verfahren aufarbeiten.

Das gewonnene freie Amin kann entweder als weiteres Wertprodukt für weitere Synthesen verwendet werden oder aber vorteilhaft nach Racemisierung in den Prozeß auf Stufe des Verfahrensschritt (d) rückgeführt werden. Auch das direkt unter Racemisierung gespaltene Amid kann vorteilhaft auf dieser Stufe in den Prozeß rückgeführt werden. Auf diese Weise kann theoretisch das gesamte Racemat in das gewünschte Enantiomer überführt werden. Solche Racemisierungen können z.B. unter denselben Bedingungen, unter denen Amine aus Alkoholen oder Ketonen hergestellt werden ("reduktive Aminierung"), durchgeführt werden. Die bei der Hydrolyse gebildete Säure kann nach Ansäuern der Hydrolyselösung vorzugsweise extraktiv zurückgewonnen und nach üblichen Verfahren verestert und rückgeführt werden.

Vorteilhaft eignen sich die erfindungsgemäßen Verfahren zur Herstellung von racemischen Aminoalkoholen der allgemeinen Formel Ic sowie zur deren Racematspaltung über enantioselektive Acylierung 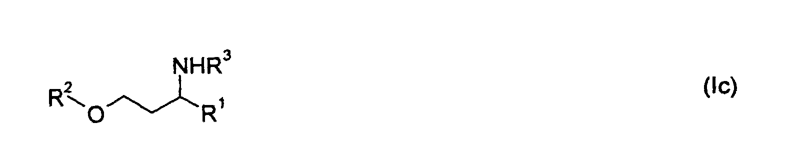 in der die Substituenten die unter Formel I genannte Bedeutung haben.

Die Erfindung betrifft weiterhin Verbindungen der Formel IX: 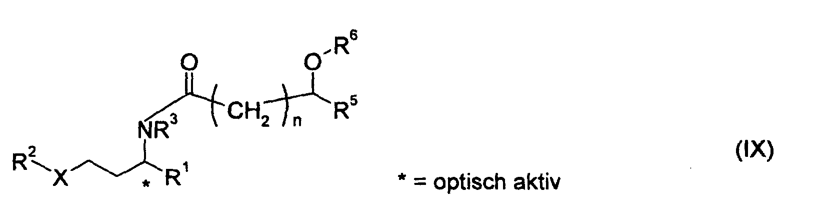 in der die Substituenten und Variablen in den Formeln IX die folgende Bedeutung haben:
R¹ bedeutet in der allgemeinen Formel IX substituiertes oder unsubstituiertes, verzweigtes oder unverzweigtes C₁-C₁₀-Alkyl-, verzweigtkettiges oder unverzweigtkettiges Phenyl-( C₁-C₅-Alkyl)-, Naphthyl-( C₁-C₅-Alkyl)-, substituiertes oder unsubstituiertes Phenyl-, Methoxyphenyl- oder Naphthyl-.
   Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl oder Propyl.
   Unter verzweigtkettige oder unverzweigtkettige Phenyl-(C₁-C₅-Alkyl)- oder Naphthyl-(C₁-C₅-Alkyl)-Reste sind Reste zu verstehen wie Phenylmethyl, Phenylethyl, Phenylpropyl, Phenyl-1-methylethyl, Phenylbutyl, Phenyl-1-methylpropyl, Phenyl-2-methylpropyl, Phenyl-1,1-dimethylethyl, Phenylpentyl, Phenyl-1-methylbutyl, Phenyl-2-methylbutyl, Phenyl-3-methylbutyl, Phenyl-2,2-dimethylpropyl, Phenyl-1-ethylpropyl, Naphthylmethyl, Naphthylethyl, Naphthylpropyl, Naphthyl-1-methylethyl, Naphthylbutyl, Naphthyl-1-methylpropyl, Naphthyl-2-methylpropyl, Naphthyl-1,1-dimethylethyl, Naphthylpentyl, Naphthyl-1-methylbutyl, Naphthyl-2-methylbutyl, Naphthyl-3-methylbutyl, Naphthyl-2,2-dimethylpropyl, oder Naphthyl-1-ethylpropyl, sowie ihre isomeren oder stereoisomeren Formen. Bevorzugte Reste sind verzweigtkettige oder unverzweigtkettige Phenyl- (C₁-C₅-Alkyl)-Reste wie wie Phenylmethyl, Phenylethyl oder Phenylpropyl.
   R¹ bedeutet weiterhin Phenyl, Methoxyphenyl oder Naphthyl, die ggf. mit einem oder mehreren Resten wie Halogen wie Fluor, Amino, Hydroxy, Alkyl, Alkoxy oder anderen Resten substituiert sein können.
   R² bedeutet in der allgemeinen Formel IX substituiertes oder unsubstituiertes, verzweigtes oder unverzweigtes C₁-C₁₀-Alkyl-, verzweigtkettiges oder unverzweigtkettiges Phenyl-( C₁-C₅-Alkyl)-, Naphthyl-( C₁-C₅-Alkyl)-, substituiertes oder unsubstituiertes Phenyl-, Methoxyphenyl- oder Naphthyl-.
   Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugte Reste sind Methyl, Ethyl oder Propyl.
   Unter verzweigtkettige oder unverzweigtkettige Phenyl-(C₁-C₅-Alkyl)- oder Naphthyl-(C₁-C₅-Alkyl)-Reste sind Reste zu verstehen wie Phenylmethyl, Phenylethyl, Phenylpropyl, Phenyl-1-methylethyl, Phenylbutyl, Phenyl-1-methylpropyl, Phenyl-2-methylpropyl, Phenyl-1,1-dimethylethyl, Phenylpentyl, Phenyl-1-methylbutyl, Phenyl-2-methylbutyl, Phenyl-3-methylbutyl, Phenyl-2,2-dimethylpropyl, Phenyl-1-ethylpropyl, Naphthylmethyl, Naphthylethyl, Naphthylpropyl, Naphthyl-1-methylethyl, Naphthylbutyl, Naphthyl-1-methylpropyl, Naphthyl-2-methylpropyl, Naphthyl-1,1-dimethylethyl, Naphthylpentyl, Naphthyl-1-methylbutyl, Naphthyl-2-methylbutyl, Naphthyl-3-methylbutyl, Naphthyl-2,2-dimethylpropyl, oder Naphthyl-1-ethylpropyl, sowie ihre isomeren oder stereoisomeren Formen. Bevorzugte Reste sind verzweigtkettige oder unverzweigtkettige Phenyl- (C₁-C₅-Alkyl)-Reste wie wie Phenylmethyl, Phenylethyl oder Phenylpropyl.
R² bedeutet weiterhin Phenyl, Methoxyphenyl oder Naphthyl, die ggf. mit einem oder mehreren Resten wie Halogen wie Fluor, Amino, Hydroxy, Alkyl, Alkoxy oder anderen Resten substituiert sein können.

Wobei die Reste R³, R⁵ und R⁶ sowie die Variablen X und n die vorgenannte Bedeutung haben.

Bevorzugte Verbindungen der Formeln I, VII und IX sind die folgenden Verbindungen:

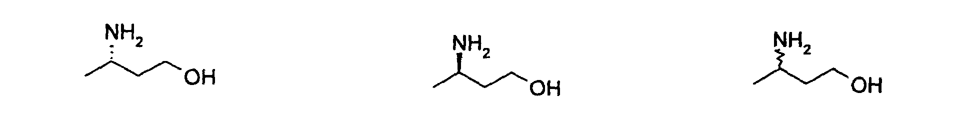

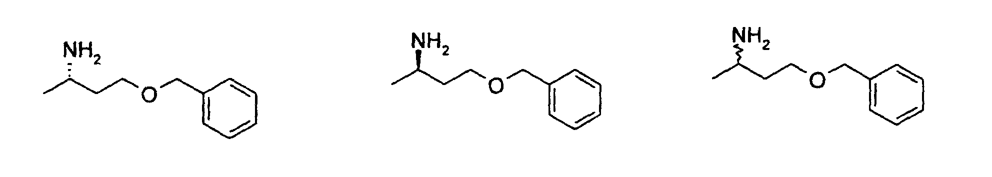

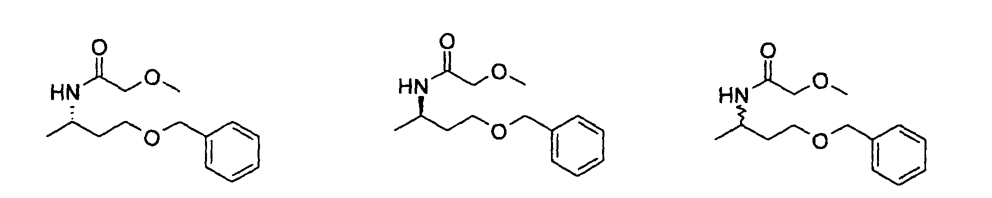

Die erfindungsgemäßen Verfahren eignen sich nicht nur als Herstellverfahren zur Produktion optisch aktiver primärer und sekundärer funktionalisierter Amine wie Aminoalkohole oder Diamine, sondern können auch Bestandteil von komplizierten chemischen Mehrstufensynthesen, beispielsweise bei der Herstellung von Arzneiwirkstoffen oder Pflanzenschutzmitteln, sein.

Die folgenden Beispiele dienen der Veranschaulichung der Erfindung.

### Beispiele

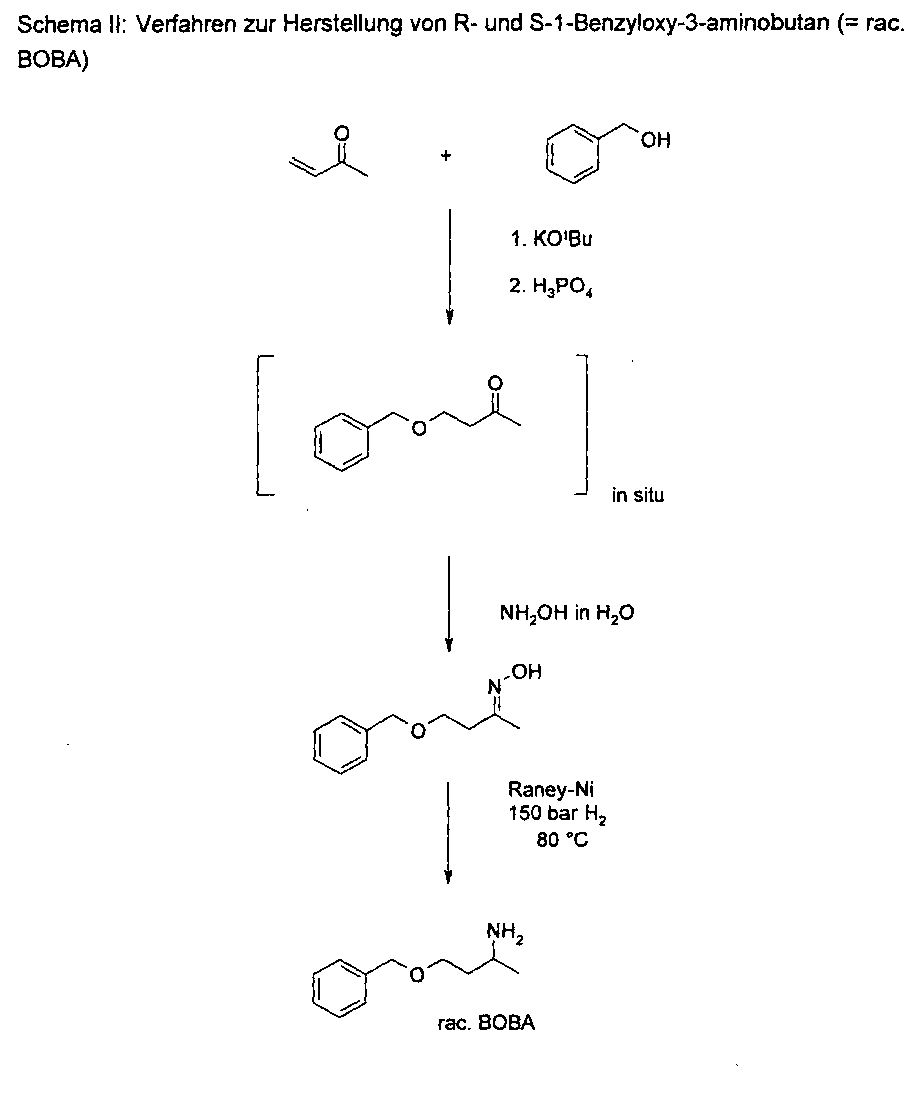

### Beispiel 1: Verfahrensschritt (a) und (b):

Synthese von 1-Benzyloxy-3-butanonoxim (Schema III)

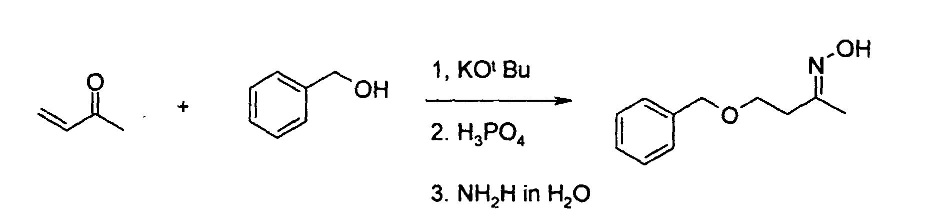

648 g (6.0 mol) Benzylalkohol wurden bei 0°C mit 2,6 g (24 mmol) Kalium-tert.butanolat versetzt und 30 Minuten gerührt. Innerhalb von 30 Minuten wurden 441 g (6.3 mol) frisch destilliertes Methyl-vinylketon zugetropft. Durch Kühlen wurde die Temperatur dabei bei 0 - 10°C gehalten. Nach beendeter Zugabe wurde noch eine Stunde bei 10°C nachgerührt. Anschließend wurden 2,8 g (24 mmol) ortho-Phosphorsäure (als 85 %-ige wäßrige Lösung) zugetropft. Das Gemisch wurde auf Raumtemperatur (= 23°C) erwärmt.
Laut. ¹H-NMR entstand bei der Reaktion 1-Benzyloxy-3-butanon (1H-NMR: δ = 2.05 (s; 3H), 2.60 (t, J= 7.0 Hz; 2H), 3.65 (t, J = 7.0 Hz; 2H), 4.45 (s, 2H), 7.25 (m, 5H).

Zu dem rohen 1-Benzyloxy-3-butanon wurde unter heftigem Rühren 471 g einer 51.7 %-igen wäßrigen Lösung (7.37 mol) von Hydroxylamin so zugetropft, so daß die Reaktionstemperatur bei ca. 35°C blieb. Anschließend wurde 15 Stunden nachgerührt. Anderntags wurde die obere, wäßrige Phase abgetrennt, die untere, organische Phase mit 500 ml Toluol aufgenommen und am Wasserabscheider erhitzt bis kein Wasser mehr mit übergeht. Das Lösungsmittel und flüchtige Bestandteile wurden bei 0.5 mm abdestilliert (Badtemperatur: 100°C), als Rückstand blieben 1100 g (95 %) 1-Benzyloxy-3-butanon-oxim als E/Z-Gemisch.

### Hauptisomer (ca. 65 %):

¹H-NMR: δ = 1.90 (s; 3H), 2.50 (t, J= 7.0 Hz; 2H), 3.65 (t, J = 7.0 Hz; 2H), 4.50 (s, 2H), 7.30 (m, 5H), 9.30 (s, breit, 1 H).

### Mindermengenisomer (ca. 35 %):

¹H-NMR: δ = 1.95 (s; 3H), 2.75 (t, J= 7.0 Hz; 2H), 3.65 (t, J = 7.0 Hz; 2H), 4.50 (s, 2H), 7.30 (m, 5H), 9.30 (s, breit, 1H).

### Beispiel 2: Hydrierung zu 1-Benzyloxy-3-aminobutan (= rac. BOBA) [Verfahrensschritt (c), Schema IV]

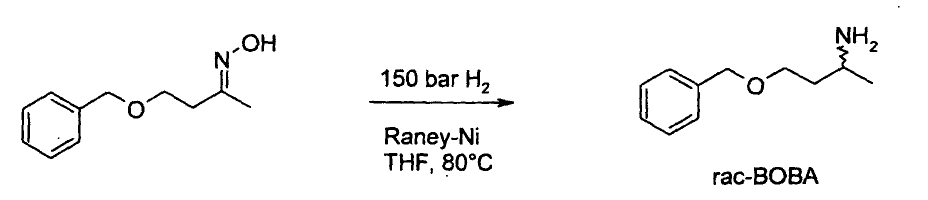

In einem Autoklaven wurden 100 g Raney-Ni#kel in 300 ml THF vorgelegt und mit einer Lösung von 850 g (4.4 mol) 1-Benzyloxy-3-butanon-oxim in 2.5 I THF versetzt. Es wurden 100 bar Wasserstoff auf den Autoklaven gestellt und unter Rühren auf 80°C aufgeheizt. Dabei setzte eine exotherme Reaktion ein, durch Nachdosieren des Wasserstoffgases wurde der Druck konstant gehalten. Nach beendeter Wasserstoffaufnahme, wurde der Druck auf 150 bar erhöht und noch 4 Stunden nachgerührt. Anschließend wurde abgekühlt, der Rührer abgestellt und vom Katalysator abdekantiert. Die leicht trübe Produktlösung wurde über Kieselgur filtriert und das klare Filtrat am Rotavapor vom Lösungsmittel befreit. Der schwerflüchtige Rückstand wurde anschließend im Ölpumpenvakuum fraktioniert destilliert. Bei 1.3 mm / 85 - 87°C geht das Produkt rac-BOBA als klare Flüssigkeit über.

Ausbeute: 646 g (83 %)

δ¹H-NMR: δ = 1.05 (d, J= 7 Hz; 3H), 1.35 (s, breit; 2H), 1.65 (mc; 2H), 3.10 (mc; 1H), 3.55 (mc; 2H), 4.50 (s, 2H), 7.30 (m, 5H).

### Beispiel 3: Racematspaltung [Verfahrensschritt (d)]

### A. Enantiomerenanalytik des BOBA:

Derivatisierung (Schema V): 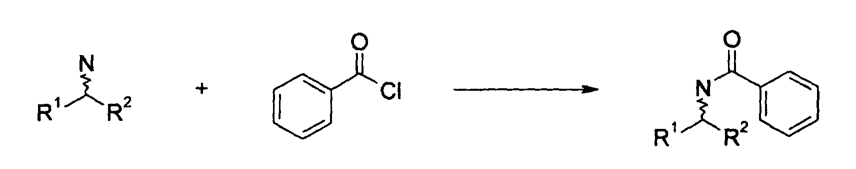 0.5 g Amin wurden in 25 ml Diethylether gelöst. Die Lösung wurde auf 0°C abgekühlt und in einem Zuge wurden 0.2 ml Benzoylchlorid zugegeben. Das Gemisch wurde bei Raumtemperatur noch 30 Minuten gerührt und dann mit 10 ml Wasser versetzt. Die wäßrige Phase wurde abgetrennt und die obere organische Phase nacheinander mit je 10 ml 10 %-iger Salzsäure, Wasser und gesättigter Natriumhydrogencarbonatlösung gewaschen. Die Reaktionslösung wurde über Natriumsulfat getrocknet. Es wurden dann 1 ml der Lösung entnommen, mit 5 ml n-Hexan verdünnt und durch HPLC-Chromatographie analysiert.

| | |
|---|---|
| Säule | Chiralcel OD |
| | Daicel Chemical Industries, Ltd. |
| Temperatur | Raumtemperatur |
| Detektor | Absorption bei 214 nm |
| Einspritzmenge | 20 µl |
| Elutionsmittel | n-Hexan / l-Propanol / Ethanol |
| | (300:50:0.8 v/v/v) |
| Fluß | 1.3 ml / Min (bei ca. 40 kg/cm² |

### Retentionszeiten:

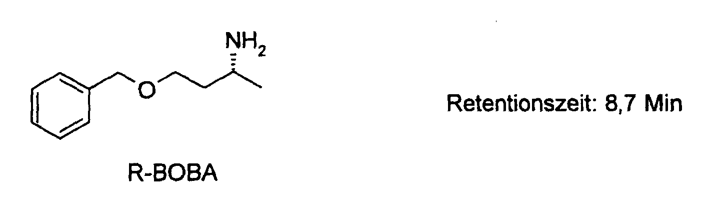

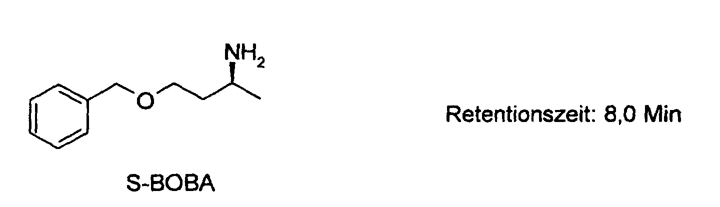

### Beispiel 4: Racematspaltung durch Kristallisation

Zu einer Lösung von 12.8 g (71.5 mmol) rac-BOBA in 70 ml iso-Propanol wurde eine Lösung von 2.15 g (35.6 mmol) Essigsäure und 5.4 g (35.6 mmol) D-Mandelsäure in 50 ml iso-Propanol getropft. Der ausgefallene Feststoff wurde durch Erhitzen wieder in Lösung gebracht und dann zum Auskristallisieren stehengelassen.

Aus einer Probe des abgesaugten Salzes wurde das gebundene Amin durch Behandlung mit Natronlauge freigesetzt.

Laut. HPLC-Analytik wies das S-BOBA einen ee-Wert von 50.5 % auf. Daraufhin wurde das ausgefallene Salz nochmals aus 100 ml iso-Propanol umkristallisiert. Anschließend wurde das Amin aus dem gebildeten Salz durch Behandlung mit 10 ml 50 %-iger Natronlauge freigesetzt, mit 50 ml Ether extrahiert und der Extrakt eingeengt. Es blieben 2.2 g (34 %) S-BOBA zurück.
Laut. HPLC-Analytik betrug die Enantiomerenreinheit 90 % ee.

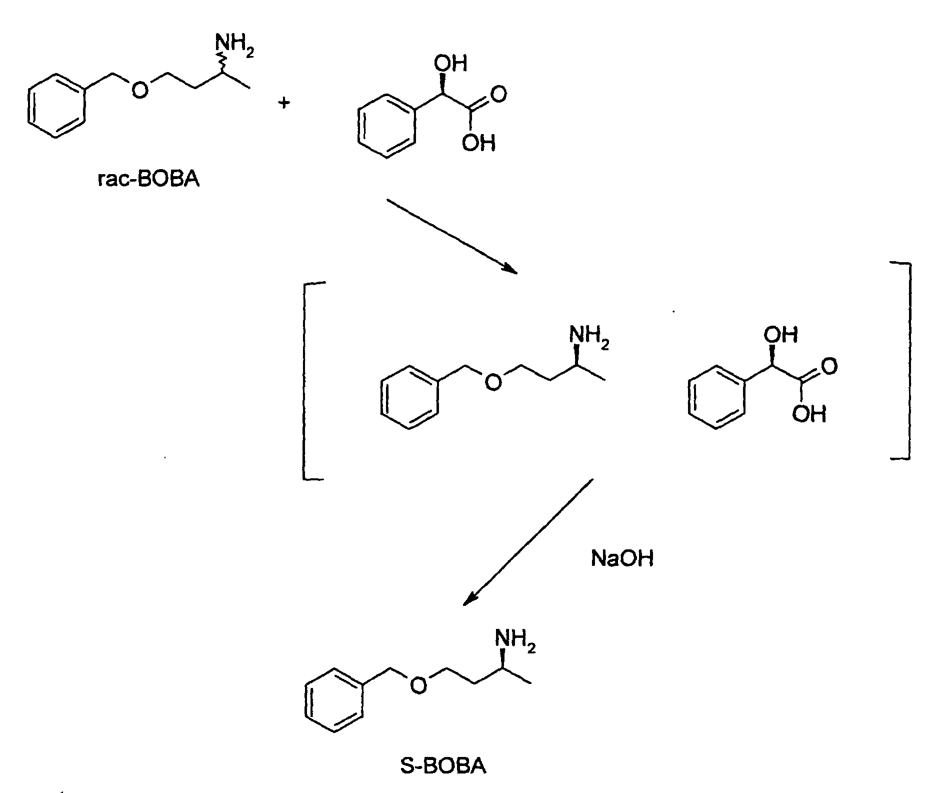

### Beispiel 5: Racematspaltung durch enzymkatalysierte Racematspaltung

500 g (8.43 mol) rac-BOBA wurden auf 0°C gekühlt und mit 523 g (3.96 mol) Methoxyessigsäure-iso-Propylester versetzt. Es wurden 75 g Novozym 435[ zugegeben und unter Rühren auf Raumtemperatur (ca. 23°C) erwärmt. Nach 15 Stunden wurde der Katalysator abfiltriert und mit 1 I Toluol gewaschen. Das Filtrat wurde im Vakuum (20 mbar) von flüchtigen Bestandteilen befreit und anschließend in einem Dünnfilmverdampfer destilliert (1.0 mbar, 180°C). Als Kopfdestillat ging S-BOBA bei (95 - 98°C) über, als Schwersieder ging R-BOBAmid in den Destillationssumpf.

Es wurden 802.5 g (53 %) S-BOBA erhalten, das laut HPLC-Analytik eine optische Reinheit von 90 % ee aufweist.

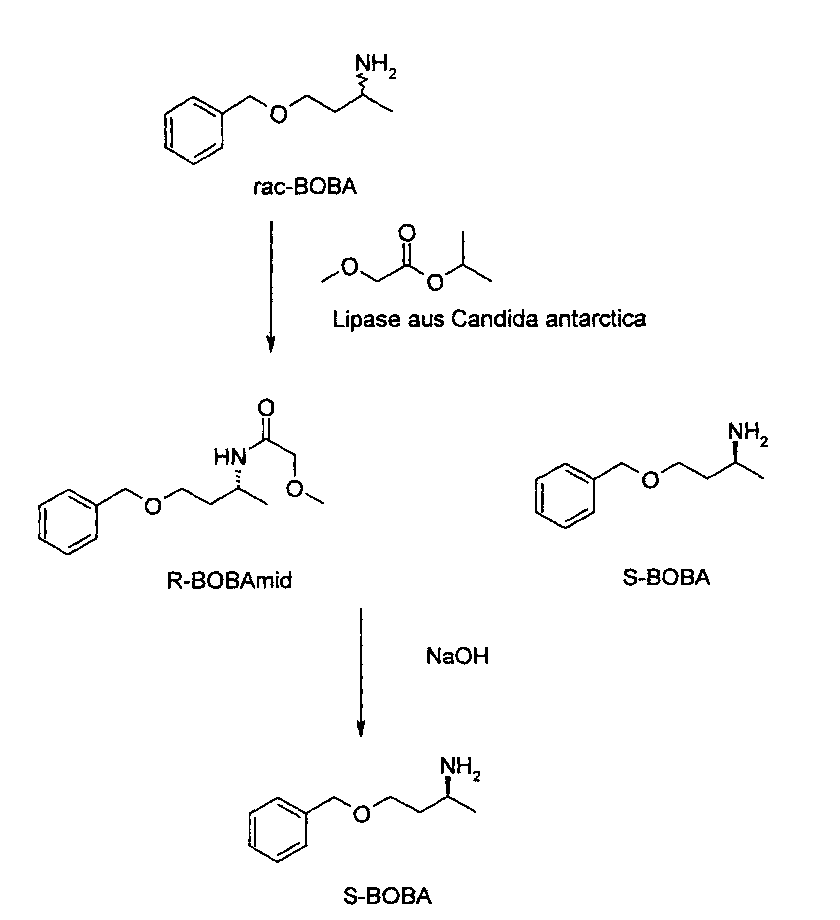

Als Sumpf fielen 964 g (45.5 %) R-BOBAmid (opt. Reinheit: 98% ee) an.

R-BOBAmid, ¹H-NMR: δ= 1.20 (d, J = 7 Hz; 3H), 1.65 - 1.95 (mc; 2H), 3.15 (s; 3H), 3.50 - 3.70 (mc; 2H), 3.75 und 3.85 (AB-System, JAB = 10.5 Hz; 2H), 4.20 (mc; 1 H), 4.50 (s, 2H), 6.90 (s, breit; 1H), 7.10 - 7.40 (m, 5H).

### Beispiel 6: Spaltung des R-BOBAmids

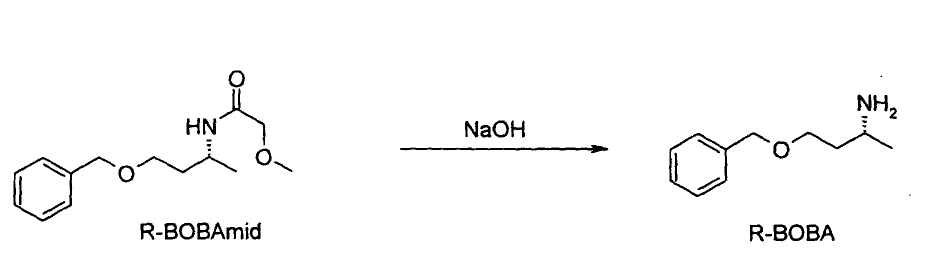

Eine Mischung aus 918 g (3.66 mol) R-BOBAmid und 900 g Triethanolamin wurde bei 120°C mit 800 g (10 mol) 50 %-iger Natronlauge versetzt und drei Stunden bei dieser Temperatur gerührt. Nach abkühlen, wurde mit 1.5 I Wasser verdünnt und dreimal mit je 1 I Diethylether extrahiert. Die vereinten Extrakte wurden nacheinander mit 1 I Wasser und 100 ml gesättigter NaCl-Lösung gewaschen, über Na₂SO₄ getrocknet und dann eingeengt. Als Rückstand blieben 625 g (95 %) R-BOBA als leicht gelbes Öl.

Die optische Reinheit betrug laut HPLC 97 % ee.

## Patentansprüche

1. Verfahren zur Herstellung von Verbindungen der allgemeinen Formel I 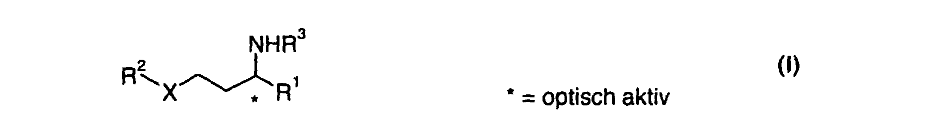 **dadurch gekennzeichnet, daß** es folgende Verfahrensschritte umfaßt:
a) Umsetzung von Verbindungen der allgemeinen Formel II 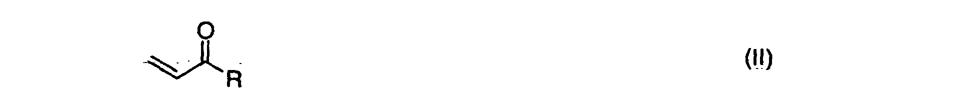 mit Verbindungen der Formel R²-XH (III) in Gegenwart einer Base zu Verbindungen der allgemeinen Formel IV 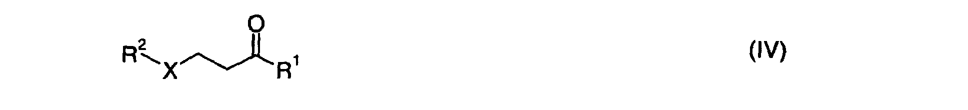
b) Umsetzung der Reaktionslösung von Verbindungen der allgemeinen Formel IV mit einer Verbindung der allgemeinen Formel NH₂R³ (V) zu Verbindungen der allgemeinen Formel VI 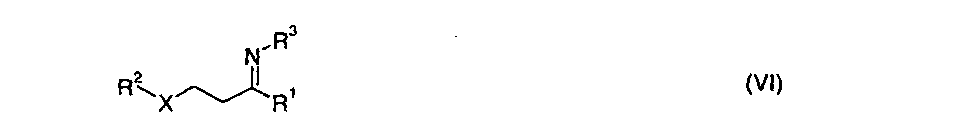
c) Hydrierung von Verbindungen der allgemeinen Formel VI zu Verbindungen der allgemeinen Formel VII 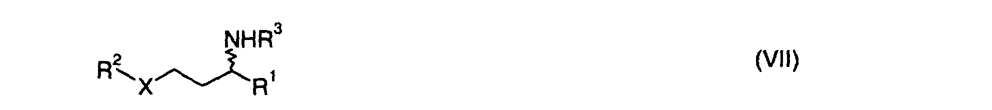 in Gegenwart eines Hydrierkatalysators,
d) Racematspaltung von Verbindungen der allgemeinen Formel VII zu Verbindungen der allgemeinen Formel I mit einer optisch aktiven Carbonsäure oder mit Estern der allgemeinen Formel VIII 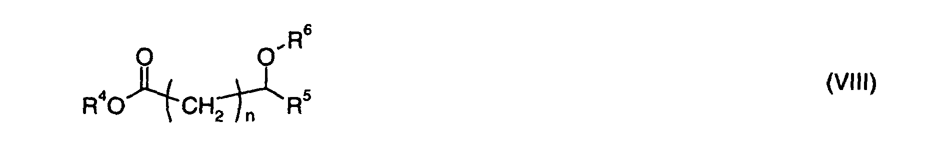 in Gegenwart einer Lipase oder Esterase, wobei die Substituenten und Variablen in den Formeln I, II, III, IV, V, VI, VII und VIII folgende Bedeutung haben:
R¹ substituiertes oder unsubstituiertes, verzweigtes oder unverzweigtes C₁-C₁₀-Alkyl-, Arylalkyl-, Aryl-, Hetarylalkyl- oder Hetaryl-,
R² substituiertes oder unsubstituiertes, verzweigtes oder unverzweigtes C₁-C₁₀-Alkyl-, Arylalkyl-, Aryl-, Hetarylalkyl- oder Hetaryl-,
R³ Wasserstoff, Hydroxyl-, substituiertes oder unsubstituiertes, verzweigtes oder unverzweigtes C₁-C₁₀-Alkyl-,
R⁴ substituiertes oder unsubstituiertes, verzweigtes oder unverzweigtes C₁-C₁₀-Alkyl-,
R⁵ Wasserstoff, substituiertes oder unsubstituiertes, verzweigtes oder unverzweigtes C₁-C₁₀-Alkyl-,
R⁶ Wasserstoff, substituiertes oder unsubstituiertes, verzweigtes oder unverzweigtes C₁-C₁₀-Alkyl- oder substituiertes oder unsubstituiertes Phenyl,
X = Sauerstoff oder Stickstoff,
n = 0 oder 1.

2. Verfahren zur Herstellung von Verbindungen der allgemeinen Formel VII 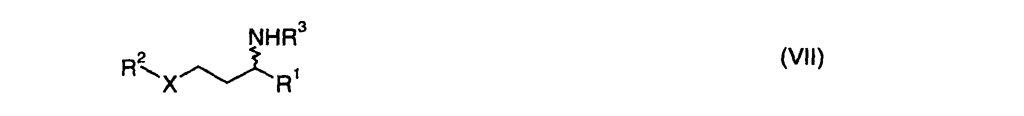 **dadurch gekennzeichnet, daß** es folgende Verfahrensschritte umfaßt:
a) Umsetzung von Verbindungen der allgemeinen Formel II 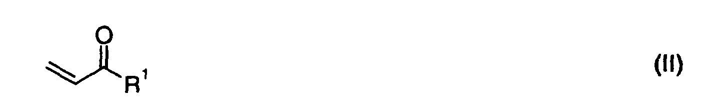 mit Verbindungen der Formel R²-XH (III) in Gegenwart einer Base zu Verbindungen der allgemeinen Formel IV 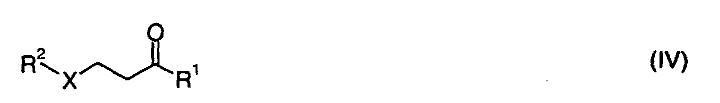
b) Umsetzung der Reaktionslösung von Verbindungen der allgemeinen Formel IV mit einer Verbindung der allgemeinen Formel NH₂R³ (V) zu Verbindungen der allgemeinen Formel VI 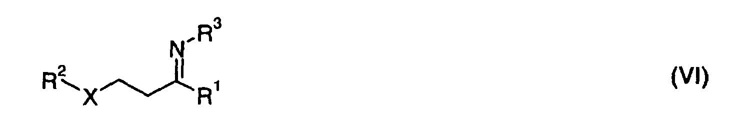
c) Hydrierung von Verbindung der allgemeinen Formel VI zu Verbindungen der allgemeinen Formel VII in Gegenwart eines Hydrierkatalysators, wobei die Substituenten und Variablen in den Formeln II, III, IV, V, VI und VII die gemäß Anspruch 1 genannte Bedeutung haben.

3. Verfahren nach Anspruch 1, **dadurch gekennzeichnet, daß** man zur Racematspaltung als optisch aktiven Carbonsäure eine Carbonsäure ausgewählt aus der Gruppe Weinsäure, Di-benzoyl-Weinsäure, Mandelsäure, Camphersäure, Campersulfonsäure, p-Hydroxy-Mandelsäure, p-Cl-Mandelsäure, Phenoxypropionsäure, p-Hydroxy-phenoxy-propionsäure oder Milchsäure verwendet.

4. Verfahren nach Anspruch 1, **dadurch gekennzeichnet, daß** man das bei der enzymkatalysierten Racematspaltung mit einem Ester unter Schritt 1 (d) erhaltene Gemisch aus optisch aktivem heteroatomsubstituierten Amin der allgemeinen Formel I und optisch aktivem heteroatomsubstituierten Amid der allgemeinen Formel IX 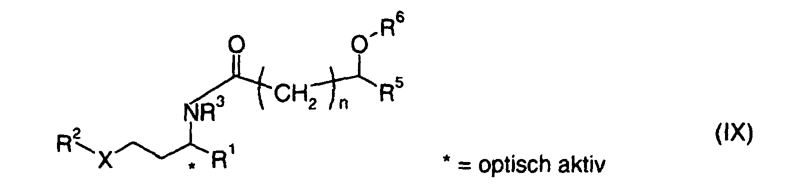 trennt, wobei die Substituenten und Variablen in der Formel IX die unter Anspruch 1 genannte Bedeutung haben.

5. Verfahren nach Anspruch 4, **dadurch gekennzeichnet, daß** man das Amid spaltet und das durch Spaltung erhaltende freie Amin gewinnt.

6. Verfahren nach Anspruch 4, **dadurch gekennzeichnet, daß** man das erhaltene Amid unter spaltenden Bedingungen racemisiert und in das Verfahren nach Anspruch 1 auf Stufe (d) zurückführt.

7. Verfahren nach Anspruch 5, **dadurch gekennzeichnet, daß** man das erhaltene Amin racemisiert und in das Verfahren nach Anspruch 1 auf Stufe (d) zurückführt.

8. Verfahren nach den Ansprüchen 1 oder 2, **dadurch gekennzeichnet, daß** man Verfahrensschritt (a) in Gegenwart eines unter Reaktionsbedingungen inerten, aprotischen Lösungsmittels durchführt.

9. Verfahren nach Anspruch 1 oder 2, **dadurch gekennzeichnet, daß** die Base in Verfahrensschritt (a) in einem Bereich von 0,001 - 10 mol% bezogen auf die eingesetzte Menge der Verbindung der Formel III verwendet wird.

10. Verfahren nach Anspruch 9, **dadurch gekennzeichnet, daß** in Verfahrensschritt (a) als Base Alkali- oder Erdalkalialkoholate oder starke basische Amine verwendet werden.

11. Verfahren nach Anspruch 1 oder 2, **dadurch gekennzeichnet, daß** man die unter Verfahrensschritt (a) erhaltene Reaktionslösung vor der Umsetzung mit einer Verbindung der allgemeinen Formel (V) neutralisiert.

12. Verfahren nach Anspruch 1 oder 2, **dadurch gekennzeichnet, daß** die Verbindungen der Formel IV, VI oder VII nach den Verfahrensschritten (a) bis (c) isoliert werden, bevor der nächste Verfahrensschritt begonnen wird.

13. Verfahren nach Anspruch 1 oder 2, **dadurch gekennzeichnet, daß** als Verbindung der allgemeinen Formel NH₂R³ Hydoxylamin verwendet wird.

14. Verfahren nach Anspruch 12, **dadurch gekennzeichnet, daß** man Verfahrensschritt (b) in Gegenwart eines unter Reaktionsbedingungen inerten, protischen Lösungsmittels durchführt.

15. Verfahren nach Anspruch 1 oder 2, **dadurch gekennzeichnet, daß** man in Verfahrensschritt (c) Hydrierkatalysatoren auf Basis Ni, Co, Pt, Pd, Ru oder Rh verwendet.

16. Verfahren nach Anspruch 14, **dadurch gekennzeichnet, daß** man als Hydrierkatalysator Raney-Nickel verwendet.

17. Verfahren nach Anspruch 13 oder 14, **dadurch gekennzeichnet, daß** die Hydrierung in einem Lösungsmittel durchgeführt wird.

18. Verfahren nach den Ansprüchen 14 bis 16, **dadurch gekennzeichnet, daß** die Hydrierung bei Normaldruck bis 300 bar durchgeführt wird.

19. Verbindungen der Formel IX 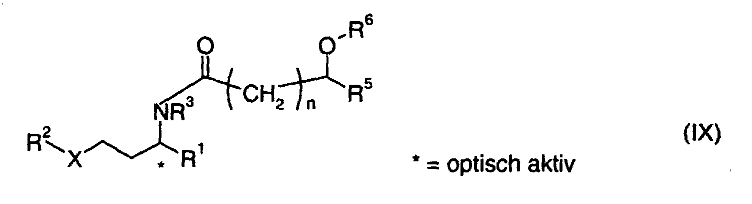 in der die Substituenten und Variablen in der Formel IX für X = O oder NH folgende Bedeutung haben:
R¹ substituiertes oder unsubstituiertes, verzweigtes oder unverzweigtes C₁-C₁₀-Alkyl-, verzweigtkettiges oder unverzweigtkettiges Phenyl-( C₁-C₅-Alkyl)-, Naphthyl-( C₁-C₅-Alkyl)-, substituiertes oder unsubstituiertes Phenyl-, Methoxyphenyl- oder Naphthyl-,
R² substituiertes oder unsubstituiertes, verzweigtes oder unverzweigtes C₁-C₁₀-Alkyl-, verzweigtkettiges oder unverzweigtkettiges Phenyl-( C₁-C₅-Alkyl)-, Naphthyl-( C₁-C₅-Alkyl)-, substituiertes oder unsubstituiertes Phenyl-, Methoxyphenyl- oder Naphthyl-,
R³ Wasserstoff, Hydroxyl-, substituiertes oder unsubstituiertes, verzweigtes oder unverzweigtes C₁-C₁₀-Alkyl-,
R⁵ Wasserstoff, substituiertes oder unsubstituiertes, verzweigtes oder unverzweigtes C₁-C₁₀-Alkyl-,
R⁶ Wasserstoff, substituiertes oder unsubstituiertes, verzweigtes oder unverzweigtes C₁-C₁₀-Alkyl- oder substituiertes oder unsubstituiertes Phenyl,
n = 0 oder 1,
und wobei R¹, R² und R³ mit Halogen, Amino, Hydroxy, Benzyloxy, Phenyl oder Benzyl und R⁵ und R⁶ mit Halogen, Cyano, Nitro, Amino, Hydroxy, Benzyloxy, Phenyl oder Benzyl substituiert sein können:

## Claims

1. A process for preparing compounds of the formula I 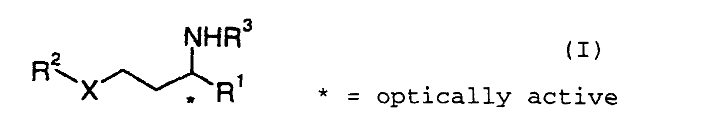 which comprises the following process steps:
a) A reaction of compounds of the formula II 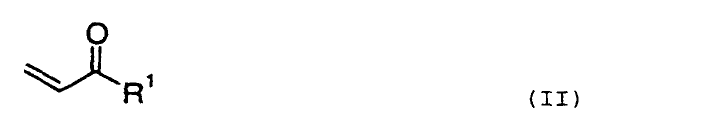 with compounds of the formula R²-XH (III) in the presence of a base to give compounds of the formula IV 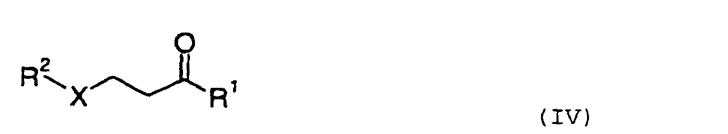
b) A reaction of the reaction solution of compounds of the formula IV with a compound of the formula NH₂R³ (V) to give compounds of the formula VI 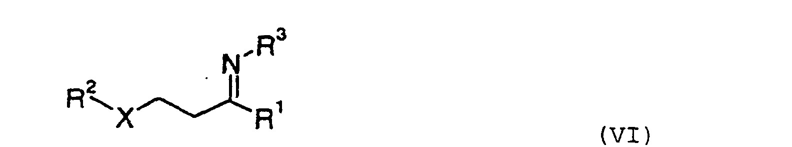
c) A hydrogenation of compounds of the formula VI in the presence of a hydrogenation catalyst to give compounds of the formula VII 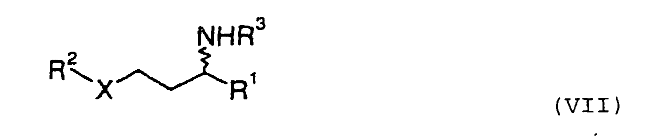
d) A resolution of a racemate of compounds of the formula VII using an optically active carboxylic acid or esters of the formula VIII 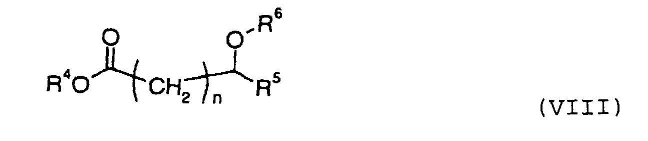 in the presence of a lipase or esterase, giving compounds of the formula I where the substituents and variables in the formulae I, II, III, IV, V, VI, VII and VIII are as defined below:
R¹ is substituted or unsubstituted, branched or unbranched C₁-C₁₀-alkyl, arylalkyl, aryl, hetarylalkyl or hetaryl,
R² is substituted or unsubstituted, branched or unbranched C₁-C₁₀-alkyl, arylalkyl, aryl, hetarylalkyl or hetaryl,
R³ is hydrogen, hydroxyl, substituted or unsubstituted, branched or unbranched C₁-C₁₀-alkyl,
R⁴ is substituted or unsubstituted, branched or unbranched C₁-C₁₀-alkyl,
R⁵ is hydrogen, substituted or unsubstituted, branched or unbranched C₁-C₁₀-alkyl,
R⁶ is hydrogen, substituted or unsubstituted, branched or unbranched C₁-C₁₀-alkyl or substituted or unsubstituted phenyl,
X = oxygen or nitrogen,
n = 0 or 1.

2. A process for preparing compounds of the formula VII 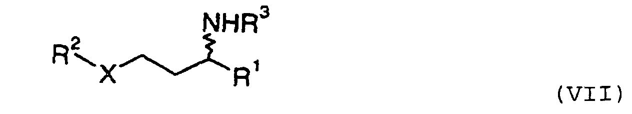 which comprises the following process steps:
a) A reaction of compounds of the formula II 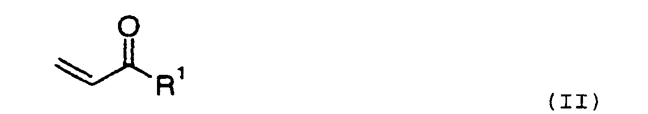 with compounds of the formula R²-XH (III) in the presence of a base to give compounds of the formula IV 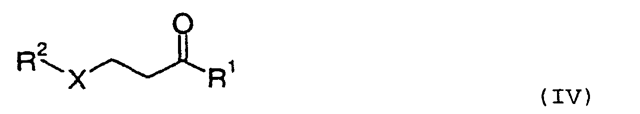
b) A reaction of the reaction solution of compounds of the formula IV with a compound of the formula NH₂R³ (V) to give compounds of the formula VI 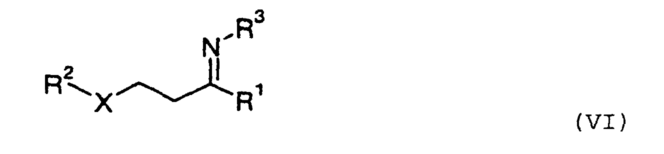
c) A hydrogenation of compounds of the formula VI in the presence of a hydrogenation catalyst to give compounds of the formula VII, where the substituents and variables in the formulae II, III, IV, V, VI and VII are as defined in claim 1.

3. A process as claimed in claim 1, wherein the optically active carboxylic acid used for the resolution of the racemate is a carboxylic acid selected from the group consisting of tartaric acid, dibenzoyltartaric acid, mandelic acid, camphoric acid, camphorsulfonic acid, p-hydroxymandelic acid, p-Cl-mandelic acid, phenoxypropionic acid, p-hydroxyphenoxypropionic acid and lactic acid.

4. A process as claimed in claim 1, wherein the mixture, obtained in step 1 (d) in the enzyme-catalyzed racemate resolution using an ester, of optically active heteroatom-substituted amine of the formula I and optically active heteroatom-substituted amide of the formula IX 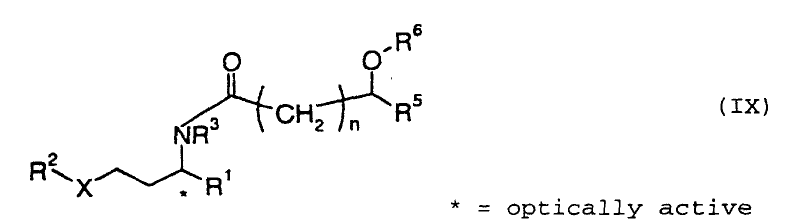 is separated, where the substituents and variables in the formula IX are as defined in claim 1.

5. A process as claimed in claim 4, wherein the amide is cleaved, giving the free amine.

6. A process as claimed in claim 4, wherein the resulting amide is racemized under cleaving conditions and recycled into step (d) of the process as claimed in claim 1.

7. A process as claimed in claim 5, wherein the resulting amine is racemized and recycled into step (d) of the process as claimed in claim 1.

8. A process as claimed in claim 1 or 2, wherein process step (a) is carried out in the presence of an aprotic solvent which is inert under the reaction conditions.

9. A process as claimed in claim 1 or 2, wherein the base in process step (a) is employed in a range from 0.001 - 10 mol%, based on the amount used of the compound of the formula III.

10. A process as claimed in claim 9, wherein the base used in process step (a) is an alkali metal alkoxide or alkaline earth metal alkoxide or a strong basic amine.

11. A process as claimed in claim 1 or 2, wherein the reaction solution obtained in process step (a) is neutralized prior to the reaction with a compound of the formula (V).

12. A process as claimed in claim 1 or 2, wherein the compounds of the formulae IV, VI and VII are isolated after process steps (a) to (c) before the next process step is started.

13. A process as claimed in claim 1 or 2, wherein the compound of the formula NH₂R³ used is hydroxylamine.

14. A process as claimed in claim 12, wherein process step (b) is carried out in the presence of a protic solvent which is inert under the reaction conditions.

15. A process as claimed in claim 1 or 2, wherein the hydrogenation catalysts used in process step (c) are based on Ni, Co, Pt, Pd, Ru or Rh.

16. A process as claimed in claim 14, wherein the hydrogenation catalyst used is Raney nickel.

17. A process as claimed in claim 13 or 14, wherein the hydrogenation is carried out in a solvent.

18. A process as claimed in any of claims 14 to 16, wherein the hydrogenation is carried out at from atmospheric pressure to 300 bar.

19. A compound of the formula IX 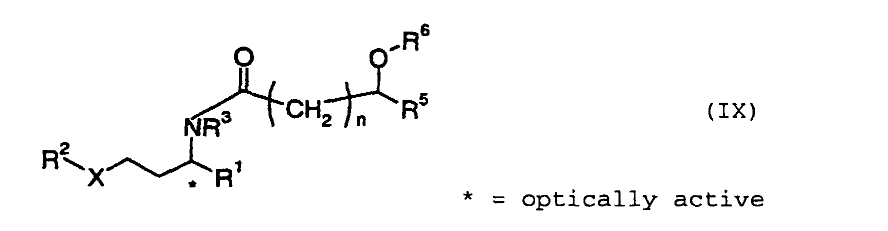 in which the substituents and variables in the formula IX for X = O or NH are as defined below:
R¹ is substituted or unsubstituted, branched or unbranched C₁-C₁₀-alkyl, branched or unbranched phenyl (C₁-C₅-alkyl), naphthyl (C₁-C₅-alkyl), substituted or unsubstituted phenyl, methoxyphenyl or naphthyl,
R² is substituted or unsubstituted, branched or unbranched C₁-C₁₀-alkyl, branched or unbranched phenyl(C₁-C₅-alkyl), naphthyl(C₁-C₅-alkyl), substituted or unsubstituted phenyl, methoxyphenyl or naphthyl,
R³ is hydrogen, hydroxyl, substituted or unsubstituted, branched or unbranched C₁-C₁₀-alkyl,
R⁵ is hydrogen, substituted or unsubstituted, branched or unbranched C₁-C₁₀-alkyl,
R⁶ is hydrogen, substituted or unsubstituted, branched or unbranched C₁-C₁₀-alkyl or substituted or unsubstituted phenyl,
n is 0 or 1,
and where R¹, R² and R³ may be substituted by halogen, amino, hydroxyl, benzyloxy, phenyl or benzyl and R⁵ and R⁶ may be substituted by halogen, cyano, nitro, amino, hydroxyl, benzyloxy, phenyl or benzyl.

## Revendications

1. Procédé de préparation de composés de formule générale I : 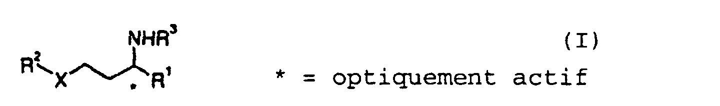 **caractérisé en ce qu'**il comporte les étapes de procédé suivantes :
a) une réaction de composés de formule générale II : 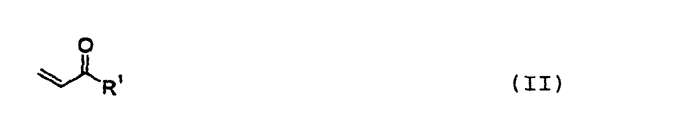 avec des composés de formule R²-XH (III), en présence d'une base, pour former des composés de formule générale IV : 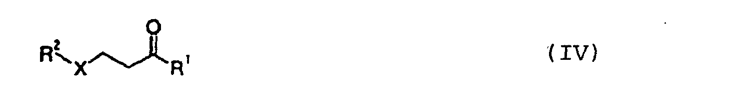
b) une réaction de la solution réactionnelle de composés de formule générale IV avec un composé de formule générale NH₂R³ (V) pour former des composés de formule générale VI : 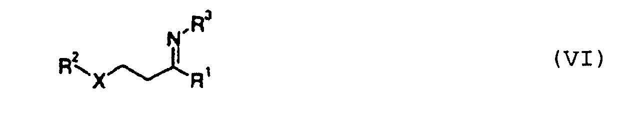
c) une hydrogénation de composés de formule générale VI en composés de formule générale VII : 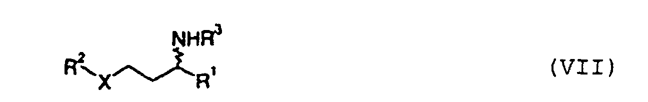 en présence d'un catalyseur d'hydrogénation,
d) un dédoublement de racémate de composés de formule générale VII en composés de formule générale I par un acide carboxylique optiquement actif ou par des esters de formule générale VIII : 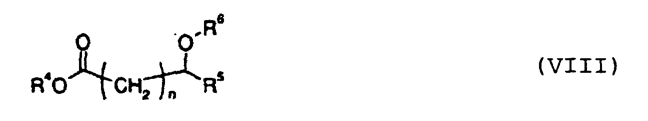
en présence d'une lipase ou d'une estérase, les substituants et variables des formules I, II, III, IV, V, VI, VII et VIII ayant la signification suivante :
R¹ est un groupe alkyle en C₁-C₁₀, arylalkyle, aryle, hétarylalkyle ou hétaryle, substitué ou non substitué, ramifié ou non ramifié,
R² est un groupe alkyle en C₁-C₁₀, arylalkyle, aryle, hétarylalkyle ou hétaryle, substitué ou non substitué, ramifié ou non ramifié,
R³ est de l'hydrogène, un groupe hydroxyle ou un groupe alkyle en C₁-C₁₀ substitué ou non substitué, ramifié ou non ramifié,
R⁴ est un groupe alkyle en C₁-C₁₀ substitué ou non substitué, ramifié ou non ramifié,
R⁵ est de l'hydrogène ou un groupe alkyle en C₁-C₁₀ substitué ou non substitué, ramifié ou non ramifié,
R⁶ est de l'hydrogène ou un groupe alkyle en C₁-C₁₀ substitué ou non substitué, ramifié ou non ramifié, ou un groupe phényle substitué ou non substitué,
X est de l'oxygène ou de l'azote,
n = 0 ou 1.

2. Procédé de préparation de composés de formule générale VII : 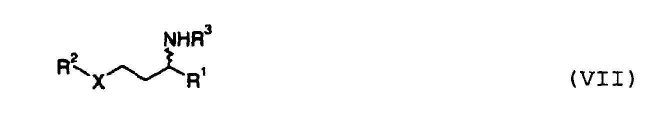 **caractérisé en ce qu'**il comporte les étapes suivantes :
a) une réaction de composés de formule générale II : 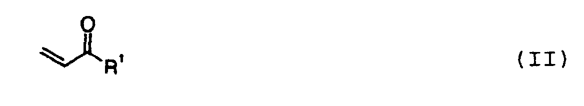 avec des composés de formule R²-XH (III), en présence d'une base, pour former des composés de formule générale IV : 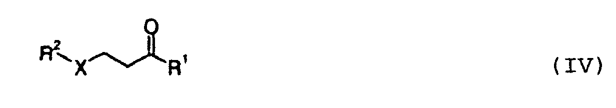
b) une réaction de la solution réactionnelle de composés de formule générale IV avec un composé de formule générale NH₂R³ (V) pour former des composés de formule générale VI : 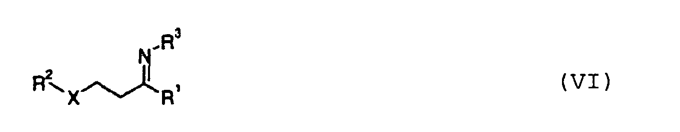
c) une hydrogénation de composés de formule générale VI en composés de formule générale VII en présence d'un catalyseur d'hydrogénation, les substituants et variables des formules II, III, IV, V, VI et VII ayant la signification donnée dans la revendication 1.

3. Procédé suivant la revendication 1, **caractérisé en ce que**, pour le dédoublement de racémate, on utilise comme acide carboxylique optiquement actif, un acide carboxylique choisi parmi le groupe de l'acide tartrique, de l'acide di-benzoyl-tartrique, de l'acide mandélique, de l'acide camphorique, de l'acide camphorsulfonique, de l'acide p-hydroxy-mandélique, de l'acide p-Cl-mandélique, de l'acide phénoxypropionique, de l'acide p-hydroxyphénoxy-propionique ou de l'acide lactique.

4. Procédé suivant la revendication 1, **caractérisé en ce que** l'on sépare le mélange d'amine optiquement active, substituée par un hétéroatome, de formule générale I et d'amide optiquement actif, substitué par un hétéroatome, de formule générale IX : 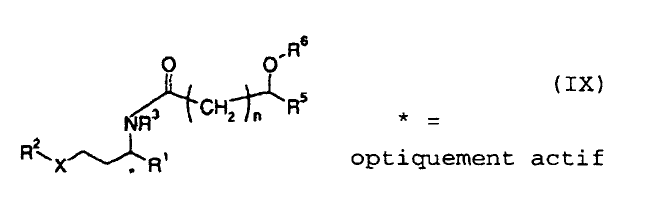 obtenu lors du dédoublement de racémate, catalysé par une enzyme, avec un ester dans l'étape 1 (d), les substituants et variables de formule IX ayant la signification donnée dans la revendication 1.

5. Procédé suivant la revendication 4, **caractérisé en ce qu'**on clive l'amide et **en ce qu'**on produit l'amine libre obtenue par clivage.

6. Procédé suivant la revendication 4, **caractérisé en ce qu'**on racémise l'amide obtenu, dans des conditions de clivage, et **en ce qu'**on le reconduit dans l'étape (d) du procédé suivant la revendication 1.

7. Procédé suivant la revendication 5, **caractérisé en ce qu'**on racémise l'amine obtenue et **en ce qu'**on la reconduit dans l'étape (d) du procédé suivant la revendication 1.

8. Procédé suivant l'une des revendications 1 et 2, **caractérisé en ce qu'**on effectue l'étape (a) du procédé en présence d'un solvant aprotique, inerte dans les conditions réactionnelles.

9. Procédé suivant l'une des revendications 1 et 2, **caractérisé en ce que** la base de l'étape (a) du procédé est utilisée dans une gamme de 0,001 à 10 moles % par rapport à la quantité mise en oeuvre du composé de formule III.

10. Procédé suivant la revendication 9, **caractérisé en ce que**, dans l'étape (a) du procédé, on utilise, comme base, des alcoolates de métal alcalin ou alcalino-terreux ou des amines basiques fortes.

11. Procédé suivant la revendication 1 ou 2, **caractérisé en ce qu'**on neutralise la solution réactionnelle obtenue dans l'étape (a) du procédé, avant la réaction avec un composé de formule générale (V).

12. Procédé suivant la revendication 1 ou 2, **caractérisé en ce que** les composés des formules IV, VI ou VII sont isolés après les étapes (a) à (c) du procédé, avant que l'on ne commence l'étape suivante du procédé.

13. Procédé suivant la revendication 1 ou 2, **caractérisé en ce qu'**on utilise de l'hydroxylamine comme composé de formule générale NH₂R³.

14. Procédé suivant la revendication 12, **caractérisé en ce qu'**on effectue l'étape (b) du procédé en présence d'un solvant protique, inerte dans les conditions réactionnelles.

15. Procédé suivant la revendication 1 ou 2, **caractérisé en ce que**, dans l'étape (c) du procédé, on utilise des catalyseurs d'hydrogénation à base de Ni, Co, Pt, Pd, Ru ou Rh.

16. Procédé suivant la revendication 14, **caractérisé en ce que**, comme catalyseur d'hydrogénation, on utilise du nickel de Raney.

17. Procédé suivant la revendication 13 ou 14, **caractérisé en ce que** l'hydrogénation est effectuée dans un solvant.

18. Procédé suivant l'une des revendications 14 à 16, **caractérisé en ce que** l'hydrogénation est effectuée à pression atmosphérique normalisée jusqu'à 300 bars.

19. Composé de formule IX : 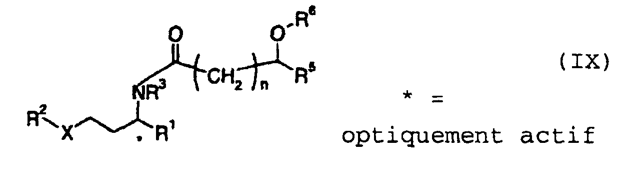 dans laquelle les substituants et variables de formule IX ont la signification suivante pour X = O ou NH :
R¹ est un groupe alkyle en C₁-C₁₀ substitué ou non substitué, ramifié ou non ramifié, phényl-(alkyle en C₁-C₅) à chaîne ramifiée ou non ramifiée, naphtyl(alkyle en C₁-C₅), phényle substitué ou non substitué, méthoxyphényle ou naphtyle,
R² est un groupe alkyle en C₁-C₁₀ substitué ou non substitué, ramifié ou non ramifié, phényl-(alkyle en C₁-C₅) à chaîne ramifiée ou non ramifiée, naphtyl(alkyle en C₁-C₅), phényle substitué ou non substitué, méthoxyphényle ou naphtyle,
R³ est de l'hydrogène, un groupe hydroxyle ou un groupe alkyle en C₁-C₁₀ substitué ou non substitué, ramifié ou non ramifié,
R⁵ est de l'hydrogène ou un groupe alkyle en C₁-C₁₀ substitué ou non substitué, ramifié ou non ramifié,
R⁶ est de l'hydrogène ou un groupe alkyle en C₁-C₁₀ substitué ou non substitué, ramifié ou non ramifié, ou phényle substitué ou non substitué,
n = 0 ou 1,
R¹, R² et R³ pouvant être substitués par de l'halogène ou un groupe amino, hydroxy, benzyloxy, phényle ou benzyle et R⁵ et R⁶ par de l'halogène ou un groupe cyano, nitro, amino, hydroxy, benzyloxy, phényle ou benzyle.
